# EUROPEAN PATENT SPECIFICATION

(11) **EP 4 069 062 B1**
(45) Date of publication and mention of the grant of the patent: **06.11.2024**
(21) Application number: 20829652.5
(22) Date of filing: 04.12.2020
(51) Int. Cl.: A61B 5/00, A61L 31/08, A61L 31/10, A61N 1/36, A61N 1/375

(54) **IMPLANTABLE STIMULATOR WITH AN ELECTRODE ARRAY AND CONFORMABLE SUBSTRATE**
IMPLANTIERBARER STIMULATOR MIT EINER ELEKTRODENANORDNUNG UND ANPASSBAREM SUBSTRAT
STIMULATEUR IMPLANTABLE DOTÉ D'UN RÉSEAU D'ÉLECTRODES ET D'UN SUBSTRAT CONFORMABLE

(30) Priority: 04.12.2019 US 201916703706; 03.04.2020 NL 2025268
(43) Date of publication of application: 12.10.2022
(73) Proprietor: Salvia Bioelectronics B.V., 5656 AE Eindhoven (NL)
(72) Inventor: MARTENS, Hubert, 5656AE Eindhoven (NL); SCHOBBEN, Daniël, 5656AE Eindhoven (NL); VAN DER ZALM, Maartje, 5656AE Eindhoven (NL); BOERE, Stijn, 5656AE Eindhoven (NL)
(74) Representative: Lees, Kate Jane
(86) International application number: PCT/IB2020/061474
(87) International publication number: WO 2021/111371

(56) References cited:
- WO-A1-2015/107515
- WO-A1-2016/033369
- WO-A2-02/096482
- US-A1- 2006 173 497
- US-A1- 2007 128 420
- US-A1- 2011 015 686
- US-A1- 2016 067 477
- VAJARI D ASHOURI ET AL: "Hybrid multimodal Deep Brain probe (DBS array) for advanced brain research", 2015 7TH INTERNATIONAL IEEE/EMBS CONFERENCE ON NEURAL ENGINEERING (NER), IEEE, 22 April 2015 (2015-04-22), pages 280 - 283, XP033166214, DOI: 10.1109/NER.2015.7146614

## Description

### COPYRIGHT NOTICE

A portion of the disclosure of this patent document contains material which is subject to copyright protection. The copyright owner has no objection to the facsimile reproduction of the patent document or the patent disclosure, as it appears in the Patent and Trademark Office patent file or records, but otherwise reserves all copyright rights whatsoever.

### TECHNICAL FIELD

The present disclosure relates to an implantable stimulator, for providing electrical stimulation to human or animal tissue, having an electrode array located along a conformable portion of a substrate, In particular, it relates to an implantable stimulator having an encapsulation layer at least partially covering a portion of the substrate. It also relates to a method of manufacturing an implantable stimulator.

### BACKGROUND

Implantable electrical stimulation systems may be used to deliver electrical stimulation therapy to patients to treat a variety of symptoms or conditions such as headaches, lower back pain and incontinence.

In many electrical stimulation applications, it is desirable for a stimulator, typically comprising a therapeutic lead (a lead comprises electrodes and electrical connections), to provide electrical stimulation to one or more precise locations within a body - in many cases, precisely aligning the stimulation electrodes during implantation may be difficult due to the curvature of tissues and anatomical structures. A mismatch in curvature of the electrode section of a lead may create unexpected and/or unpredictable electrical resistance between one or more electrodes and the underlying tissue. In addition, repeated movement of the relevant areas of the body may even worsen the mismatch. A particular problem with subcutaneous implants is that even small differences in flexibility between the implant and surrounding tissue may affect patient comfort, and can cause irritation of the overlying skin. This is a particular problem with sub-cutaneous implants.

In particular, the use of neurostimulation leads in the craniofacial region is associated with skin erosion and lead migration. The cylindrical shape and associated thickness of state-of-the-art leads results in the lead eroding through the skin or results in the lead being displaced so that the electrodes no longer cover the targeted nerves.

More recently, use has been made of plastics and polymers, which have an inherent flexibility or may be made in a curved shape - for example, as described in US application US 2016/0166828. Although such leads may be manufactured in a curved-shape or deformed by human manual manipulation during implantation, this is inconvenient. The high degree of anatomic variability found in humans and animals means that a manufacturer must provide either a large range or pre-curved leads or allow the leads to be made to measure. In the case that they are deformable during implantation, this further complicates the implantation process.

Implantable active devices require a protection method to protect the implant electronics from bodily fluids present in human or animal bodies. Bodily fluids typically contain ions that may cause electrochemical reactions, like corrosion, in the presence of an electric current. Encapsulation is thus a critical component for the design of a medical device - it acts as a barrier between these ionic fluids and critical electronic/electric interfaces to reduce and/or prevent degradation of the implant electronics.

Polyimides are popular for use as a substrate material for the microfabrication of electronics, and attempts have been made to encapsulate polyimides with silicone rubber encapsulants, such as polydimethylsiloxane rubber (PDMS). As described in "Irreversible bonding of polyimide and polydimethylsiloxane (PDMS) based on a thiol-epoxy click reaction", Hoang, Chung and Elias, Journal of Micromechanics and Microengineering, 10.1088/0960-1317/26/10/105019, bonding these two flexible materials remains a crucial challenge - the resistance to fluid ingress may be reduced by the encapsulant delaminating to some degree from the substrate. The degree of bonding was increased by functionalizing the surfaces of the PDMS and polyimide substrates with mercaptosilanes and epoxysilanes, respectively, for the formation of a thiolepoxy bond in the click reaction. It was also increased by functionalizing one or both surfaces with mercaptosilane and introducing an epoxy adhesive layer between the two surfaces.

Although PDMS can be substantially biocompatible, causing minimal tissue reaction while having a relative long period of biostability, it still has a relatively high permeability to moisture which can lead to degradation of the implant electronics. Many other encapsulants with a lower degree of moisture permeability may have a lower degree of biocompatibility. Recently, LCP's (Liquid Crystal Polymers) have been considered for use as a substrate for electronics, and there is also a need for improved bonding techniques between LCP and encapsulants.

US2016/067477 describes a neurostimulation system with three main parts: an implantable pulse generator, a paddle lead body, and a flexible paddle electrode array. An enclosure extends about the entirety of all surfaces of the electrode array, and also the pulse generator is enclosed in a sealed portion for protection of the components.

### SUMMARY

It is to be understood that both the following summary and the detailed description are exemplary and explanatory and are intended to provide further explanation of the invention as claimed. Neither the summary nor the description that follows is intended to define or limit the scope of the invention to the particular features mentioned in the summary or in the description. Rather, the scope of the invention is defined by the appended claims. Embodiments in the description relating to methods of treatment are not covered by the claims. Any "embodiment" or "example" which is disclosed in the description but is not covered by the claims should be considered as presented for illustrative purpose only.

An implantable stimulator is provided, comprising: a pulse generator (500) configured to generate at least one stimulation pulse; a conformable foil-like substrate (300, 1400) having a longitudinal axis (600) extending from the pulse generator (500) to a distal end of the substrate (300, 1400), the substrate (300, 1400) comprising one or more adjacent polymeric substrate layers, the substrate having a first (310, 1410) and second (320, 1420) substantially planar surface; and an electrode array (200, 400, 1220), proximate the distal end (300, 1400), having a first (200, 1220) and second (400, 1220) electrode comprised in the first (310, 1410) or second surface (320, 1420), located along the conformable portion of the substrate (300, 1420), each electrode (200, 400, 1220) in operation being configurable for transferring treatment energy, in use, to and/or from human or animal tissue; the implantable stimulator (100, 101, 102, 103, 104, 105, 1110, 1111) further comprising: one or more electrical interconnections (250, 1210), between the pulse generator (500) and the first (200, 1220) and the second (400, 1220) electrodes, for transferring electrical energy as one or more electrical treatment stimulation pulses to the coupled first electrodes (200) and/or the second electrodes (400); wherein the one or more electrical interconnections (250, 1210) are positioned between the first (310, 1410) and second (320, 1420) surfaces of the substrate (300, 1400); wherein the conformable foil-like substrate (300, 1400) has a maximum thickness of 0.5 millimeter or less, proximate the first and second electrodes (200, 400, 1220), the thickness being determined by a perpendicular distance between corresponding points on the first (310, 1410) and second outer planar surfaces (320, 1420), and wherein the substrate (300, 1400) and the pulse generator (500) are embedded in one or more flexible bio-compatible encapsulation layers comprising Polydimethylsiloxane (PDMS).

The products and methods described herein provide a high degree of conformability as well as high degree of configurability. A higher degree of conformability may increase the comfort for the user. Optionally, the thickness of the conformable portion is equal to or less than 0.3 millimeters, or equal to or less than 0.2 millimeters, or equal to or less than 0.1 millimeters.

Encapsulation may improve the reliability and/or lifetime of the implantable substrate.

Additionally or alternatively, the implantable stimulator further comprises an adhesion layer adjacent to at least part of the substrate. Optionally, the substrate comprises more than one adjacent substrate layer and the adhesion layer is between substrate layers.

One or more adhesion layers may improve the performance of the encapsulation. This may also improve the reliability and/or lifetime of the implantable substrate. By providing a multilayer, thinner leads may be used, adding to the flexibility and therefore improving conformability.

Optionally, the adhesion layer comprises a ceramic material. A ceramic material may be advantageous comprised in an adhesion layer between a substrate material and encapsulant material.

Optionally, the ceramic material is selected from the group consisting of: HfO2, Al2O3, Ta2O3, SiC, Si3N4, TiO2, and any combination thereof.

Additionally or alternatively, the adhesion layer comprises at least one first layer comprising HfO2 and at least one second layer adjacent to the at least one first layer and comprising Al2O3. Additionally or alternatively, the adhesion layer comprises at least one first layer comprising Ta2O3 and at least one second layer adjacent to the at least one first layer and comprising Al2O3. Additionally or alternatively, the adhesion layer comprises at least one first layer comprising TiO2 and at least one second layer adjacent to the at least one first layer and comprising Al2O3.

Optionally, a ceramic portion of the adhesion layer has an average thickness in the range of 25nm to 200nm. Optionally, the adhesion layer comprises a ceramic portion that is applied using atomic layer deposition (ALD).

Additionally or alternatively, the thickness of the stimulator along the further portion is equal to or less than 5 millimeters, or equal to or less than 4 millimeters, or equal to or less than 3 millimeters.

This may further improve conformability of the further portion of the substrate.

Additionally or alternatively, the plurality of electrical interconnections are positioned between the first and second surfaces of the substrate using metallization. Additionally or alternatively, the plurality of electrical interconnections are comprised in one or more conductive interconnection layers, the one or more conductive interconnection layers being comprised between two adjacent polymeric substrate layers.

By providing a more easily patternable substrate, more complicated electrode array configurations may be supported, allowing a higher degree of flexibility tc address transverse and/or longitudinal misalignment.

Such products described herein provide improved bonding to improve resistance to fluid ingress in implantable devices comprising flexible substrates. The encapsulant/adhesion layer may be optimized to protect a surface of many types of substrates. As the substrate is configured and arranged to be substantially flexible, the substrate has a high degree of conformability. The high degree of adhesion of the encapsulant/adhesion layer allows the flexible encapsulant layer to provide a high degree of ingress protection for one or more surfaces of a flexible substrate. Each encapsulant/adhesion layer may be optimized separately or together to a predetermined degree.

Optionally, the conformable portion of the substrate comprises a liquid crystal polymer (LCP).

According to the claimed invention, the encapsulation layer comprises Polydimethylsiloxane (PDMS).

By providing a bilayer having an encapsulant comprising a PDMS and a conformal adhesion layer comprising ceramic materials, the adhesion layer appears to show significantly higher stability in ionic media, thereby providing relatively longer protection in case of any delamination or water permeation through the encapsulant. A PDMS may further contribute to longer-lasting adhesion and defect reduction due to flowing in-between any defects and crevices in the adhesion layer - in particular, a PDMS with a relatively low viscosity may provide an even higher degree of defect reduction.

The ceramic materials HfO2, Al2O3, Ta2O3, SiC, Si3N4, TiO2, and any combination thereof, may be advantageously used as in an adhesion layer for a PDMS encapsulant layer.

### BRIEF DESCRIPTION OF THE DRAWINGS

Certain illustrative embodiments illustrating organization and method of operation, together with objects and advantages may be best understood by reference to the detailed description that follows, taken in conjunction with the accompanying drawings, which are not necessarily drawn to scale.

The accompanying drawings, which are incorporated herein and form a part of the specification, illustrate exemplary embodiments and, together with the description, further serve to enable a person skilled in the pertinent art to make and use these embodiments and others that will be apparent to those skilled in the art:
FIG. 1A is a transverse view of a first implementation of an implantable stimulator consistent with certain embodiments of the present invention.
FIG. 1B is a top view of a first implementation of an implantable stimulator consistent with certain embodiments of the present invention.
FIG. 1C is a bottom view of a first implementation of an implantable stimulator consistent with certain embodiments of the present invention.
FIG. 2A is a transverse view of a second implementation of an implantable stimulator consistent with certain embodiments of the present invention.
FIG. 2B is a top view of a second implementation of an implantable stimulator consistent with certain embodiments of the present invention.
FIG. 2C is a bottom view of a second implementation of an implantable stimulator consistent with certain embodiments of the present invention.
FIG. 3A is a transverse view of a third implementation of an implantable stimulator consistent with certain embodiments of the present invention.
FIG. 3B is a top view of a third implementation of an implantable stimulator consistent with certain embodiments of the present invention.
FIG. 3C is a bottom view of a third implementation of an implantable stimulator consistent with certain embodiments of the present invention.
FIG. 4A is a first view of alternative electrode configurations of an implantable stimulator consistent with certain embodiments of the present invention.
FIG. 4B is a second view of alternative electrode configurations of an implantable stimulator consistent with certain embodiments of the present invention.
FIG. 4C is a third view of alternative electrode configurations of an implantable 30 stimulator consistent with certain embodiments of the present invention.
FIG. 5 presents locations of nerves in the anterior portion of a human head that may be treated through operation of an implantable stimulator consistent with certain embodiments of the present invention.
FIG. 6 presents locations of nerves in the posterior portion of a human body that may be treated through operation of an implantable stimulator consistent with certain embodiments of the present invention.
FIG. 7 presents locations of nerves in a human body that may be treated through operation of an implantable stimulator consistent with certain embodiments of the present invention.
FIG. 8A and 8B depict Electrochemical Impedance Spectroscopy (EIS) as Bode plot results of FIG. 8A: impedance magnitude and FIG. 8B: phase angle for three samples.
FIG. 8C and 8D depict EIS results at 10⁻² Hz over four hundred and fifty days of soaking as FIG. 8C: impedance magnitude and FIG. 8D: phase angle for four samples.
FIG. 9 presents measurement results comparing the average pull force, dry and after soaking, of LCP coated with PDMS using different processes.
FIG. 10 depicts a cross-section through a test sample.
FIG. 11A, FIG. 11B and FIG. 11C depict cross-sections through improved implantable electrical devices.
FIG. 12A and FIG. 12B depict cross-sections through improved implantable medical devices comprising an improved implantable electrical device, and one or more electrodes.

### DETAILED DESCRIPTION

While this invention is susceptible of embodiment in many different forms, there is shown in the drawings and will herein be described in detail specific embodiments, with the understanding that the present disclosure of such embodiments is to be considered as an example of the principles and not intended to limit the invention to the specific embodiments shown and described. The embodiments described, and their detailed construction and elements, are merely provided to assist in a comprehensive understanding of the invention. The scope of the invention is defined by the appended claims. In the description below, like reference numerals are used to describe the same, similar or corresponding parts in the several views of the drawings or even in different drawings.

Thus, it is apparent that the present invention can be carried out in a variety of ways, and does not require any of the specific features described herein. Also, well-known functions or constructions are not described in detail since they would obscure the invention with unnecessary detail. Any signal arrows in the drawings/figures should be considered only as exemplary, and not limiting, unless otherwise specifically noted,

It will be understood that, although the terms first, second, etc. may be used herein to describe various elements, these elements should not be limited by these terms. These terms are only used to distinguish one element from another. For example, a first element could be termed a second element, and, similarly, a second element could be termed a first element, without departing from the scope of example embodiments. As used herein, the term "and/or" includes any and all combinations of one or more of the associated listed items. As used herein, "at least one of A, B, and C" indicates A or B or C or any combination thereof. As used herein, the singular form of a word includes the plural, and vice versa, unless the context clearly dictates otherwise.

The terms "a" or "an", as used herein, are defined as one or more than one. The term "plurality", as used herein, is defined as two or more than two. The term "another", as used herein, is defined as at least a second or more. The terms "including" and/or "having", as used herein, are defined as comprising (i.e., open language). The term "coupled", as used herein, is defined as connected, although not necessarily directly, and not necessarily mechanically.

It should also be noted that in some alternative implementations, the functions/acts noted may occur out of the order noted in the figures. For example, two figures shown in succession may in fact be executed substantially concurrently or may sometimes be executed in the reverse order, depending upon the functionality/acts involved.

As used herein, ranges are used herein in shorthand, so as to avoid having to list and describe each and every value within the range. Any appropriate value within the range can be selected, where appropriate, as the upper value, lower value, or the terminus of the range.

The words "comprise", "comprises", and "comprising" are to be interpreted inclusively rather than exclusively. Likewise the terms "include", "including" and "or" should all be construed to be inclusive, unless such a construction is clearly prohibited from the context. The terms "comprising" or "including" are intended to include embodiments encompassed by the terms "consisting essentially of" and "consisting of'. Similarly, the term "consisting essentially of" is intended to include embodiments encompassed by the term "consisting of'. Although having distinct meanings, the terms "comprising", "having", "containing' and "consisting of" may be replaced with one another throughout the description of the invention.

"About" means a referenced numeric indication plus or minus 10% of that referenced numeric indication. For example, the term "about 4" would include a range of 3.6 to 4.4. All numbers expressing quantities of ingredients, reaction conditions, and so forth used in the specification are to be understood as being modified in all instances by the term "about." Accordingly, unless indicated to the contrary, the numerical parameters set forth herein are approximations that can vary depending upon the desired properties sought to be obtained. At the very least, and not as an attempt to limit the application of the doctrine of equivalents to the scope of any claims, each numerical parameter should be construed in light of the number of significant digits and ordinary rounding approaches.

Wherever the phrase "for example," "such as," "including" and the like are used herein, the phrase "and without limitation" is understood to follow unless explicitly stated otherwise.

"Typically" or "optionally" means that the subsequently described event or circumstance may or may not occur, and that the description includes instances where said event or circumstance occurs and instances where it does not.

Reference throughout this document to "one embodiment", "certain embodiments", "an embodiment" or similar terms means that a particular feature, structure, or characteristic described in connection with the embodiment is included in at least one embodiment of the present invention. Thus, the appearances of such phrases or in various places throughout this specification are not necessarily all referring to the same embodiment. Furthermore, the particular features, structures, or characteristics may be combined by a person skilled in the art in any suitable manner in one or more embodiments without limitation.

In the following detailed description, numerous non-limiting specific details are given to assist in understanding this disclosure. Only the embodiments that fall within the scope of the present claims are according to the claimed invention. The other embodiments are presented for illustrative purposes.

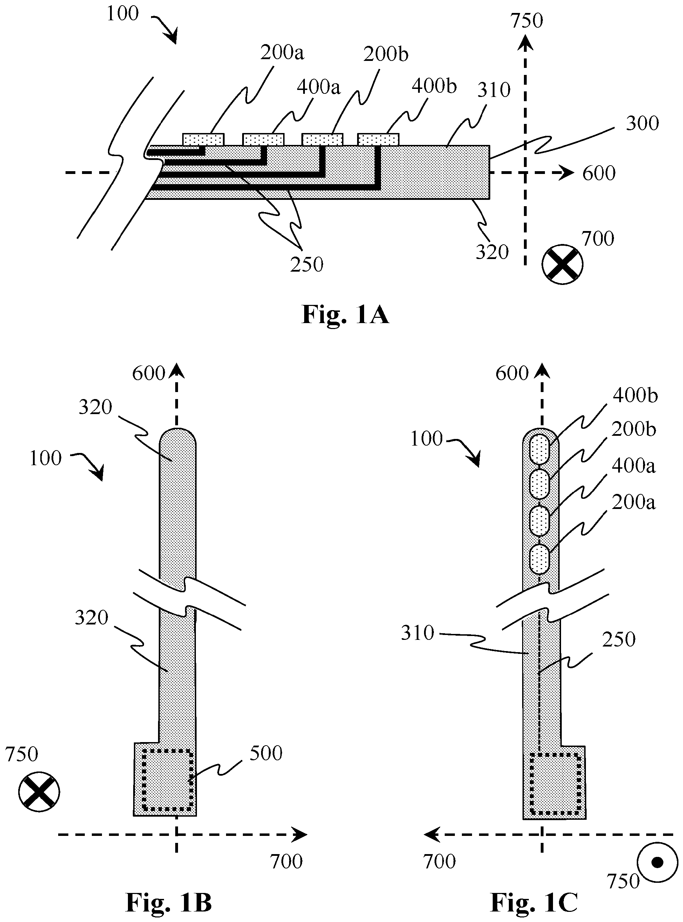

FIG. 1A, 1B & 1C depict longitudinal cross-sections through a first embodiment 100 of an implantable stimulator comprising:
- a pulse generator 500 (only depicted in FIG. 1B and 1C) for generating at least one electrical treatment stimulation pulse; and
- a conformable portion of a foil-like substrate 300 having a longitudinal axis 600 extending from the pulse generator 500 to a distal end of the substrate 300. The substrate 300 comprises one or more adjacent polymeric substrate layers and has a first 310 and a second 320 planar (outer) surface.

The implantable stimulator 100 also comprises:
- an electrode array 200, 400, proximate the distal end, having at least one electrode of a first 200a, 200b type and at least one electrode of a second type 400a, 400b. The electrodes 200, 400 are comprised in the first 310 or second 320 surface, and each is configurable for transferring treatment energy, in use, to (as a stimulation electrode) and/or from (as a return electrode) human or animal tissue. In this context, an array may be considered a systematic arrangement of two or more electrodes 200a, 200b, 400a, 400b. 1D, 2D or 3D arrays may be provided. Optionally, they may be arranged in rows and/or columns.

The implantable stimulator 100 further comprises:
- one or more electrical interconnections 250, between the pulse generator 500 and the first 200a, 200b and the second 400a, 400b electrodes, for transferring electrical energy as one or more electrical treatment stimulation pulses to the coupled first electrodes 200a, 200b and/or the second electrodes 400a, 400b. The one or more electrical interconnections 250 are comprised (or positioned) between the first surface 310 and the second 320 surfaces. A plurality of electrical interconnections 250 is considered to be two, or more than two, electrical interconnections 250.

In this disclosure, the conformability of the electrode array 200, 400 is determined to a high degree by the one or more of the following:
- the conformability of a portion of the substrate 300 proximate the electrodes 200, 300;
- the arrangement and positions of the electrodes 200, 400;
- the materials and dimensions (or extent) of the materials comprised in the electrodes 200, 400;
- the arrangement and positions of the one or more interconnections 250 proximate the electrodes 200, 400; and
- the materials and dimensions (or extent) of the materials comprised in the interconnections 200, 400.

By suitable configuration, arrangement and optimization, an implantable electrode array 200, 400 may be provided which is foil-like (or film-like) and highly conformable.

As depicted, the conformable portion of the foil-like substrate 300 is preferably elongated along the longitudinal axis 600, having a tape-like shape, allowing the pulse generator 500 to be disposed (or located) further away from the position of the electrodes 200, 400.

If the substrate 300 is substantially planar (which is according to the claimed invention, by allowing the substrate 300 to conform to a planar surface), the first 310 and second 320 surfaces are disposed along substantially parallel transverse planes 600, 700. As depicted in FIG. 1A, the first surface 310 lies in a plane comprising the longitudinal axis 600 and a first transverse axis 700 - the first transverse axis 700 is substantially perpendicular to the longitudinal axis 600. As depicted in FIG. 1A, the plane of the first surface 310 is substantially perpendicular to the plane of the cross-section drawing (substantially perpendicular to the surface of the paper).

The conformable portion of the foil-like substrate 300 has a maximum thickness of 0.5 millimeter or less, proximate the first 200a, 200b and second 400a, 400b electrodes, the thickness being defined by the first 310 and second surfaces 320 - it may be determined by a perpendicular distance between corresponding points on the first 310 and second planar surfaces 320. This is preferably determined when the substrate 300 conforms to a planar surface.

The foil-like substrate 300 has a thickness or extent along a second transverse axis 750 - this second transverse axis 750 is substantially perpendicular to both the longitudinal axis 600 and the first transverse axis 700 - it lies in the plane of the drawing (along the surface of the paper) as depicted. The first surface 310 is depicted as an upper surface and the second surface 320 is depicted as a lower surface.

The thickness may therefore be determined by a perpendicular distance along the second transverse axis 750 between corresponding points on the first 310 and second planar surfaces 320. The maximum thickness of the conformable portion of the foil-like substrate 300 proximate the first 200a, 200b and second 400a, 400b electrodes is 0.5mm or less, preferably 0.3 millimeters or less, even more preferably 0.2 millimeters or less, yet more preferably 0.1 millimeters or less.

In general, the lower the maximum thickness (in other words, the thinner the substrate), the higher the degree of conformance. However, a higher maximum thickness may be preferred to improve mechanical strength.

To clarify the differences between the different views depicted, the axes are given nominal directions:
- the longitudinal axis 600 extends from the proximal end (not depicted in FIG. 1A, but depicted in FIG. 1B and 1C) on the left, to the distal end, depicted on the right of the page;
- the first transverse axis 700 extends into the page as depicted; and
- the second transverse axis 750 extends from bottom to top as depicted.

The conformable portion of the foil-like substrate 300 may be configured and arranged as a multilayer - it comprises two or more adjacent polymeric substrate layers secured to each other, and having the first 310 and second 320 planar surface. The one or more electrical interconnections 250 are also comprised (or positioned) between the first 310 and second 320 planar surfaces. However, it is not necessary that the two or more polymeric layers and /or interconnections have similar extents along the first transverse axis 700. In other words, within the context of this disclosure, there may be regions where an interconnection 250 is sandwiched between regions of polymeric substrate (appears as a multilayer in a longitudinal cross-section), adjacent to regions where the polymeric substrate is substantially contiguous. Similarly, there may be regions where an interconnection 250 is sandwiched between two polymeric substrate layers (appears as a multilayer in a longitudinal cross-section), adjacent to regions where the substrate comprises two adjacent substrate layers. Similarly, a substrate comprising two or more polymeric substrate layer may be modified (physically and/or chemically), such that it appears to be one layer of polymeric substrate.

These polymeric substrate layers are selected for suitability to be conformable, and to comprise the one or more electrical interconnections 250. Preferably, the polymeric substrate materials are also biocompatible and durable, such as a material selected from the group comprising silicone rubber, siloxane polymers, polydimethylsiloxanes, polyurethane, polyether urethane, polyetherurethane urea, polyesterurethane, polyamide, polycarbonate, polyester, polypropylene, polyethylene, polystyrene, polyvinyl chloride, polytetrafluoroethylene, polysulfone, cellulose acetate, polymethylmethacrylate, polyethylene, and polyvinylacetate. Suitable polymer materials, including LCP (Liquid Crystal Polymer) films, are described in "Polymers for Neural Implants", Hassler, Boretius, Stieglitz, Journal of Polymer Science: Part B Polymer Physics, 2011, 49, 18-33 (DOI 10.1002/polb.22169), In particular, Table 1 is included here as reference, depicting the properties of Polyimide (UBE U-Varnish-S), Parylene C (PCS Parylene C), PDMS (NuSil MED-1000), SU-8 (MicroChem SU-8 2000 & 3000 Series), and LCP (Vectra MT1300).

Conformable foil-like substrates 300 are configured to follow the contours of the underlying anatomical features very closely by being flexible. Very thin foil-like substrates 300 have the additional advantage that they have increased flexibility.

Most preferably, the polymeric substrate layers comprise an LCP, Parylene and/or a Polyimide. LCP's are chemically and biologically stable thermoplastic polymers which allow for hermetic sensor modules having a small size and low moisture penetration.

Advantageously, an LCP may be thermoformed allowing complex shapes to be provided. Very thin (and subsequently very conformable) and very flat (highly planar) layers of an LCP may be provided. For fine tuning of shapes, a suitable laser may also be used for cutting.

In a non-limiting example, a conformable foil-like substrate 300 of LCP may have a thickness (extent along the second transverse axis 750) in the range 50 microns (um) to 720 microns (um), preferably 100 microns (um) to 300 microns (um). In an exemplary embodiment, values of 150 um (micron), 100um, 50um, or 25um may be provided.

When conforming to a substantially planar surface, the foil-like surface 300 is substantially comprised in a plane with a transverse extent substantially perpendicular to the longitudinal axis 600, wherein the planar width may be determined by a perpendicular distance between corresponding points on outer surfaces edges of the planar foil-like substrate 300 along the transverse extent. As depicted, this is along the first transverse axis 700. In an embodiment, electrode 200, 400 widths of 2 mm to 20 mm may be provided using LCP.

At room temperature, thin LCP films have mechanical properties similar to steel. This is important as implantable substrates 300 should be strong enough to be implanted, strong enough to be removed (explanted) and strong enough to follow any movement of the neighboring anatomical features and/or structures without deteriorating.

LCP belongs to the polymer materials with the lowest permeability for gases and water. LCP's can be bonded to themselves, allowing multilayer constructions with a homogenous structure.

In contrast to LCP's, polyimides are thermoset polymers, which require adhesives for the construction of multilayer portions with electrode arrays. Polyimides are thermoset polymer material with high temperature and flexural endurance.

In an embodiment, an LCP may be used to provide a conformable substrate 300 as a multilayer - in other words, two or more adjacent polymeric substrate layers. In a non-limiting example, these may be layers of 25 um (micron) thickness.

In an embodiment, one or more electrical interconnections 250 may be provided (or positioned) between the first 310 and second 320 surfaces by metallization. These may be conductors embedded in the substrate 300 such as by having a single polymer layer and applying conductive material using suitable deposition techniques known from the semiconductor industry.

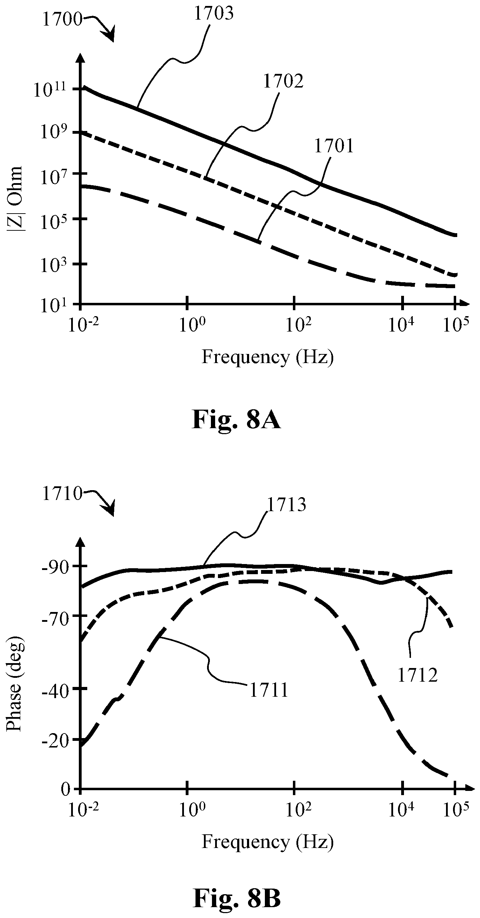

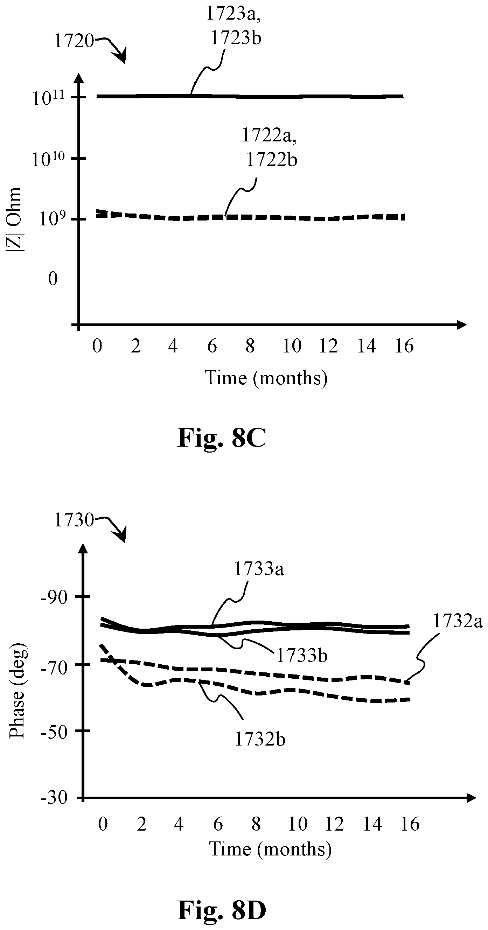

In an embodiment, if two or more adjacent polymeric substrate layers are provided, an interconnection layer may be provided using suitable techniques such as those from the semiconductor industry. The polymeric substrate layers may also be considered adjacent when one of more adhesion layers are used between them. Examples of suitable adhesion materials and adhesion layers are described below in relation to FIG. 8 to FIG. 12.

In an embodiment, lamination may also be used to provide a substrate 300 with the desired physical and chemical properties, and/or to provide a convenient method of manufacture. In a non-limiting example, a substrate 300 may comprise three laminated polymer layers: two high temperature thermoplastic layers with a low-temperature layer (bond-ply) in between, and high-temperature layers towards the first surface 310 and second surface 320.

In an alternative embodiment, two layers of silicone may be provided as polymeric substrate layers: one layer of silicone is provided, metal is patterned on one of its outer surfaces, and a second layer of silicone is added over the metal patterning by jetting, over-molding, or spin-coating.

In an embodiment, the electrical interconnections 250 may comprise one or more conductive materials, such as a metal, formed as required in one or more conductive elements: wire, strand, foil, lamina, plate, and/or sheet. They may be a substantially contiguous (one conductor). They may also comprise more than one conductor, configured and arranged to be, in use, electrically connected with each other - in other words, the one or more conductors are configured and arranged to be substantially electrically contiguous in use.

Alternatively, the one or more electrical interconnections 250 may be comprised in one or more conductive interconnection layers 250, the one or more conductive interconnection layers being comprised (or positioned) between two adjacent polymeric substrate layers. As depicted in FIG. 1A, a plurality of interconnections may be provided at different dispositions (or depths or positions) between the first surface 310 and the second surface 320.

In an embodiment, an interconnection 250 in the context of this disclosure is not configured or arranged to be, in use, in contact with human or animal tissue. The one or more interconnections 250 are embedded (or covered) in one or more layers of a low conductance or insulating polymer, such as LCP. Additionally or alternatively, one or more encapsulation layers may be used.

One or more interconnection layers 250 may also be provided by metallization using techniques from the PCB (Printed Circuit Board) industry, such as metallization with a bio-compatible metal such as gold or platinum. Electro-plating may be used. Layers comprising LCP films are particularly suitable for metallization. These electrical interconnections 250 and/or interconnect layers 250 are configured to transfer electrical energy as one or more electrical treatment stimulation pulses from the pulse generator 500 to the coupled first electrodes 200a, 200b and/or the second electrodes 400a, 400b.

Using suitable polymeric substrate materials, such as an LCP film, allows the conformable portions of the foil-like (or film-like) substrate 300 and electrode array 200, 300 to have a high width-to-height ratio, providing a bio-compatible electronic foil (or film), or bio-electronic foil (or film).

In an embodiment according to the claimed invention, i.e. when the substrate 300 conforms to a substantially planar surface, the ratio of maximum planar width to maximum thickness proximate the first 200a, 200b and second 400a, 400b electrodes may be 7:1 or higher, preferably 10:1 or higher, more preferably 15:1 or higher, yet more preferably 30:1 or higher, even more preferably 50:1 or higher.

Ratios of 100:1 or higher may also be advantageous, and may be provided using one or more mechanically strong substrate layers of an LCP film, with a width of approximately 20mm and a thickness of approximately 0.2 mm. This provides a high degree of flexibility, and therefore also a high degree of conformability. Additional measures may also be taken to increase the degree of conformability in the first transverse direction 700, such as varying the width of the substrate, adding one or more undulations and/or providing bending points.

In a non-limiting example, when using a single row of electrodes 200, 400 and/or electrodes 200, 400 with a smaller width, the width may be four mm with a thickness of approximately 0.2mm - this is a ratio of approximately 20:1.

In a non-limiting example, in a portion of the substrate proximate the pulse generator 500, greater extents may be required which further depend, to a high degree, on the dimensions of the electronic components used a width of twenty mm and a thickness of three mm. This is a ratio of approximately 6.67: 1.

As depicted in FIG. 1A, the distal end (or distal portion) of the conformable foil-like substrate 300 comprises:
- two electrodes 200a, 200b of a first type, comprised in the first surface 310, and
- two electrodes 400a, 400b of a second type, also comprised in the first surface 310. From proximal to distal end, the order depicted is 200a, 400a, 200b, 400b - in other words, each electrode of the first type 200a, 200b is proximate an electrode of the second type 400a, 400b and comprised in the same surface 310.

The foil-like substrate 300 comprises an electrical interconnection 250 between each electrode 200a, 400a, 200b, 400b and the pulse generator. In this embodiment, each electrical interconnection 250 is configured and arranged such that each electrode 200a, 400a, 200b, 400b is electrically connected substantially independently - consequently, one of the operating modes available by suitably configuring the pulse generator 500 is substantially independent operation. The pulse generator 500 may be configured using one or more hardware, firmware and/or software parameters.

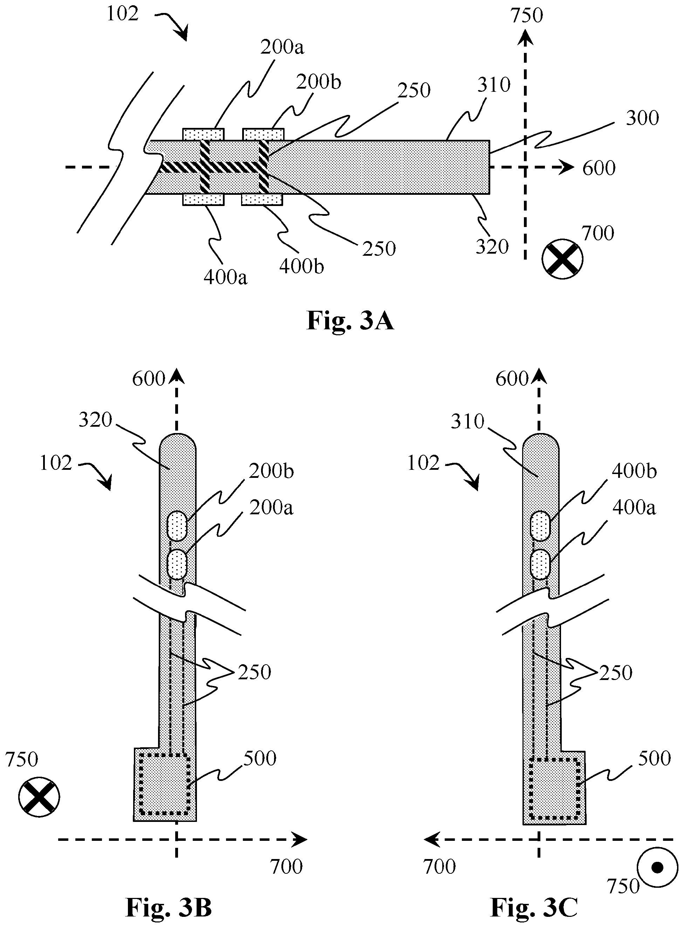

Although depicted in FIG. 1A as individual connections 250 at different distances (or positions) between the first 310 and second 320 surfaces, the skilled person will also realize that the same interconnections may be provided by a suitably configured interconnections 250 (or an interconnection layer 250) at approximately the same distance (or position) between the first 310 and second 320 surfaces, similar to the embodiment depicted in FIG. 3B, and described below.

"Comprised in" the first 310 or second 320 surface means that the electrodes 200a, 400a, 200b, 400b are relatively thin (such as when the substrate is arranged to conform to a substantially planar surface, it may have an extent along the second transverse axis of 20 to 50 microns or less. Thinner electrodes may be also be used to further increase the degree of conformability, such as 1 micron or less), and attached to (or at least partially embedded in) the surface.

The electrodes 200, 400 may comprise a conductive material such as gold, platinum, platinum black, TiN, IrO₂, iridium, and/or platinum/iridium alloys and/or oxides. Conductive polymers, such as Pedot, may also be used. Preferably, bio-compatible conductive materials are used. PCB/metallization techniques may be used to manufacture them on or in the first 310 and/or second 330 surfaces of the one or more polymeric substrate layers.

Thicker metal layers are generally preferred over thinner metal layers for electrodes 200a, 200b, 400a, 400b because they can be subjected to bodily substances that may dissolve the metal. However, thicker metal layers typically increase rigidity (reduce conformability) proximate the thicker layer.

The stimulator 100 may be implanted by first creating a subcutaneous tunnel and/or using an implantation tool. However, the high degree of conformability may make successful implantation more difficult. Even when using a suitable insertion tool, the electrode positions may be found later to be incorrect due to misalignment, lead migration during implantation, or lead migration after transplantation.

At least the distal end comprising the electrode array 200, 400, is implanted. However, it may be advantageous to implant the stimulator 100.

In addition, during implantation, it may be difficult to precisely identify the desired position for the stimulation. When implanted, the stimulator electrodes should be positioned sufficiently close to the nerve to be stimulated. But nerve pathways may not always be clearly visible to the professional performing the implantation, and the disposition and path of the nerve pathways vary greatly from person-to-person.

As depicted in FIG. 1, there is no substantial hardware difference between the first-type 200a, 200b and second type 400a, 400b electrodes - any difference in functionality is determined in this implementation mainly by the configuration (one or more hardware, firmware and/or software parameters) of the pulse generator 500. There may be a smaller influence on the electrical properties due to the arrangement and routing of the interconnections 250.

One or more coupled electrodes of the same type 200a, 200b or 400a, 400b may be operated substantially the same by suitable configuration of the pulse generator 500 - in other words, the stimulation energy applied to the electrodes 200, 400 is substantially the same at substantially the same time instance (usually measured as a voltage, a current, a power, a charge, or any combination thereof). This may also be used to anticipate and/or correct for a misalignment and/or lead migration - this is advantageous as it allows the configuration to be performed at least partially using software.

Additionally or alternatively, two or more electrodes 200, 400 may be configured and arranged using one or more parameters of the pulse generator 500 as a stimulation electrode or a return electrode. This may provide a higher degree of configurability as it only becomes necessary to implant the substrate 300 such that at least two of the electrodes are proximate the desired stimulation location.

In this embodiment 100, the electrodes of the first type 200a, 200b are nominally configured and arranged to be operated as a stimulation electrode.

The electrodes of the second type 400a, 400b are nominally configured to be operated as a return electrode - each is configured to provide, in use, an electrical return for one or more stimulation electrode 200a, 200b. In other words, the electrical return 400a, 400b closes the electrical circuit. It may also be similarly configured to provide an electrical ground for a corresponding electrical energy source.

Three configurations are thus provided based on this nominal configuration: either:
- a stimulation / return electrode pair 200a / 400a proximate the first surface 310 at that stimulation / return location; or
- a stimulation / return electrode pair 200b / 400b proximate the first surface 310 at that stimulation / return location; or

. a combination thereof.

In an embodiment, one or more stimulation electrodes 200a, 200b may be provided in such a stimulator 100. The number, dimensions and/or spacings of the stimulating electrodes 200a, 200b may be selected and optimized depending on the treatment. In an embodiment, if more than one stimulation electrode 200a, 200b is provided, each stimulation electrode 200a, 200b may provide:
- a different stimulation effect, a similar stimulation effect or the same stimulation effect.

To avoid a misalignment, a selection may be made of one or two electrodes 200a, 200b proximate the tissues where the effect is to be created.

Two or more stimulation electrodes 200a, 200b may be made active at substantially the same time if stimulation over a larger area is required and/or at a location between the active stimulation electrodes 200a, 200b.

In an embodiment, a stimulation electrode 200a, 200b may have dimensions in the order of six to eight mm along the longitudinal axis 600, and three to five mm along the first transverse axis 700, so approximately 18 to 40 square mm (mm²).

In an embodiment, a foil-like substrate 300, suitable for an implantable stimulator, may comprise up to twelve stimulation 200a, 200b and return 400a, 400b electrodes over a length of 15cm to allow for a correction for misalignment, or to simply allow the specialist to select the most effective stimulation location.

In an embodiment, FIG. 1B depicts a view of the second surface 320 of the implantable distal end (or portion) of the foil-like substrate 300 depicted in FIG. 1A. In other words, the second surface 320 is depicted in the plane of the paper, lying along the longitudinal axis 600 (depicted from bottom to top) and in the first transverse axis 700 (depicted from left to right). The second transverse axis 750 extends into the page. The first surface 310 is not depicted in FIG. 1B, but lies at a higher position along the second transverse axis 750 (into the page), and is also substantially parallel to the plane of the drawing. The foil-like substrate 300 is arranged to conform to a substantially planar surface.

The pulse generator 500 may be disposed (or positioned) between the second 320 surface and the first 310 surface. In FIG. 1B and 1C, it is depicted with dotted lines. Alternatively, the pulse generator 500 may be at least partially disposed on the first surface 310 or on the second surface 320. Alternatively, the pulse generator 500 may be at least partially embedded in the first surface 310 or in the second surface 320.

Depending on the degree of embedding and the one or more electrical components used for the pulse generator 500, the maximum thickness may be optimized. Components may be thinned to minimize the thickness. If the substrate 300 is configured and arranged to be conformable and/or foil-like, the maximum thickness of the implantable stimulator 100 in a portion of the substrate proximate the pulse generator 500 may be five millimeters or less, preferably four millimeters or less, even more preferably three millimeters or less, the thickness being determined by a perpendicular distance between corresponding points on outer planar surfaces when the implantable stimulator 100 conforms to a substantially planar surface. Additional optional electrical components, such as an antenna, comprising a coil or dipole or fractal antenna, may also influence the thickness depending on the degree that they are embedded in the substrate.

The stimulator 100 and the foil-like substrate 300 extend along the first transverse axis 700 (considered the planar width of the stimulator 100 / foil-like substrate 300 when conforming to a substantially planar surface). As depicted, the planar width in a portion of the substrate proximate the pulse generator 500 may be greater than the planar width in another portion of the substrate proximate the electrodes 200a, 200b, 400a, 400b at the distal end (or portion) of the foil-like substrate 300. The planar width proximate the pulse generator 500 may depend on the hardware and components used for the pulse generator 500 - typically, it is at least the width of the integrated circuit used for the pulse generator 500. Additional optional electrical components, such as an antenna comprising a coil or dipole or fractal antenna, may also influence the planar width.

In an embodiment, the planar width proximate the electrodes 200a, 200b, 400a, 400b may depend on the conductors used for the electrodes 200a, 200b, 400a, 400b and the one or more interconnections 250. In an embodiment, the planar width is at least the width of the first electrode 200a, 200b or the second electrode 400a, 400b.

In an embodiment, FIG. 1C depicts a view of the first surface 310 of the implantable distal end (or portion) of the foil-like substrate 300 depicted in FIG. 1A and 1B. In other words, the first surface 310 is depicted in the plane of the paper, lying along the longitudinal axis 600 (depicted from bottom to top) and in the first transverse axis 700 (depicted from right to left). The second transverse axis 750 extends out of the page. This is the view facing the animal or human tissue which is stimulated (in use). The second surface 320 is not depicted in FIG. 1C, but lies at a lower position along the second transverse axis 750 (into the page), and is also substantially parallel to the plane of the drawing. The foil-like substrate 300 is arranged to conform to a substantially planar surface.

The one or more interconnections 250 are disposed (or positioned) between the first 310 surface and the second 320 surface, as depicted in FIG. 1A. In FIG. 1C, they are depicted as dotted lines, representing the interconnections 250 (or suitably configured one or more interconnection layers 250) that have been provided for each of the electrodes 200a, 200b, 400a, 400b in this embodiment. A single dotted line 250 is depicted between the pulse generator 500 and the electrodes 200, 400 to indicate, in embodiment 100, that the interconnections 250 are at approximately the same disposition along the first transverse axis 700.

As depicted in FIG. 1C, the electrodes 200a, 200b, 400a, 400b each have a longitudinal extent (length) along the longitudinal axis 600 and a transverse extent (width) along the first transverse axis 700.

Although depicted as similar, in practice, each electrode 200a, 200b, 400a, 400b may vary in shape, transverse cross-section, orientation and/or size (or extent), depending on the intended use and/or the desired degree of configurability.

After implantation of the stimulator 100, or at least of the distal end (or portion) comprising the electrode array 200, 400, the pulse generator 500 may be configured and arranged to provide, in use, electrical energy to the one or more coupled electrodes of the first type 200a, 200b with respect to the electrical return applied to the one or more coupled electrodes of the second type 400a, 400b.

The configurability of the stimulator 100 allows, before, during and/or after implantation of at least of the distal end (or portion) comprising the electrode array 200, 400, the operation of the one or more electrodes 200a, 200b, 400a, 400b to be determined and/or adapted. The operation may also be reconfigured one or more times during the period that the stimulator 100 is implanted to optimize and/or prolong treatment.

In an embodiment, the pulse generator 500 may be initially configured to nominally operate 200a and 400a as respectively a stimulation / return electrode pair. After implantation of at least the distal end 200, 400, insufficient stimulation may be observed and/or measured. If it is assumed to be due to a mainly longitudinal misalignment, the pulse generator 500 may be alternatively configured, using one or more parameters, to nominally operate 200b and 400b as respectively a stimulation / return electrode pair.

The stimulator 100 may be further configured and arranged to switch the pulse generator 500 under predetermined and/or controlled conditions between these configurations. It may be convenient to further consider these configurations as a first and second electrode modes, and allow a user to select a mode as a preference and/or switch mode. Alternatively, the pulse generator 500 may switch modes under predetermined and/or controlled conditions.

Additionally or alternatively, other modes may also be provided for configuring the pulse generator 500 to operate in:
- a first electrode mode, wherein electrical stimulation energy is provided to one or more coupled electrodes of the first type 200a, 200b as one or more electrical treatment stimulation pulses, the one or more coupled electrodes of the second type 400a, 400b being configured to provide, in use, a corresponding electrical return for the one or more first electrodes 200a, 200b; or
- a second electrode mode, wherein energy is provided to one or more coupled electrodes of the second type 400a, 400b as one or more electrical treatment stimulation pulses, the one or more coupled electrodes of the first type 200a, 200b being configured to provide, in use, a corresponding electrical return for the one or more second electrodes 400a, 400b.

Again, the stimulator 100 may be further configured and arranged to switch the pulse generator 500 under predetermined and/or controlled conditions between these configurations or modes. Additionally or alternatively, a user may be allowed to select a mode as a preference and/or switch mode.

The skilled person will realize that the electrodes 200a, 200b, 400a, 400b may be configured to operate in more complex configurations, such as:
- 400a and 200a may be operated as respectively a stimulation / return electrode pair (reversing the original intended operation);
- 400b and 200b may be operated as respectively a stimulation / return electrode pair;
- if an intermediate stimulation is preferred, two or more electrodes 200a, 200b, 400a, 400b may be operated substantially simultaneously as one or more stimulation electrodes;
- one or more electrodes 200a, 200b, 400a, 400b may be operated as one or more return electrodes;
- electrode 400a operated as a stimulation electrode, in combination with electrode 200a and electrode 200b as return electrodes;
- electrode 400a and 200b operated as a stimulation electrode, in combination with electrode 200a and electrode 400b as a return electrode.

Alternatively or additionally, the shape, orientation, transverse cross-section, and/or size (or length) of one or more stimulation electrodes may be differently configured compared to one or more return electrodes.

A number of parameters and properties may be considered when configuring and arranging a portion of the foil-like substrate 300 proximate the electrode array 200, 400 for conformability, such as:
- the transverse 700 and/or longitudinal extent 600 of the one or more electrodes 200a, 200b, 400a, 400b
- the thickness of the foil-like substrate 300, or the perpendicular distance between the first surface 310 and the second surface 320
- the materials comprised in the foil-like substrate 300, and their physical properties
- the number and extent of interconnections 250 and/or interconnection layers 250 between the first surface 310 and second surface 320.

There have been attempts to make traditional leads, such as cylindrical leads, much thinner to allow subcutaneous implantation and/or to increase comfort by flattening. But the surface area of the flattened electrodes may become disadvantageously small.

In a non-limiting example, a conventional 0.2mm round lead with 1 cm long electrodes is estimated to result in an electrode with approximately 6 mm² electrode surface.

However, using the conformable electrode arrays described herein, a thin substrate 300 with dimensions of 0.2 mm thick, and four mm wide may be configured and arranged to provide approximately 35 mm² electrode surface in the same length. It is estimated that this may reduce impedance by a factor of approximately 35/6, and reduce power consumption by approximately 35/6.

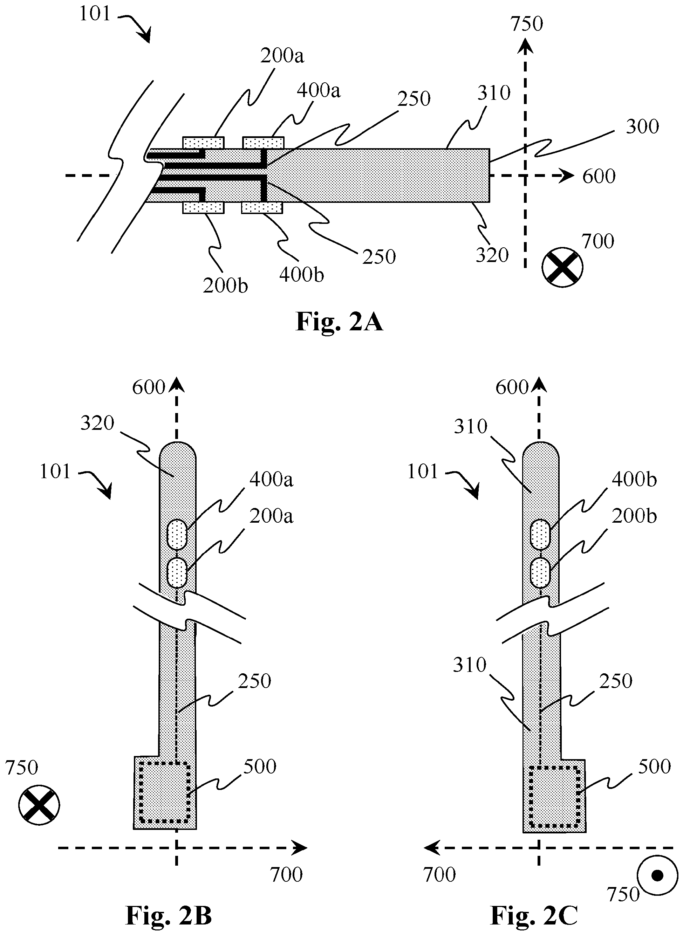

In an embodiment, FIG. 2A, 2B and 2C depict longitudinal cross-sections through a second embodiment 101 of an implantable stimulator. It is similar to the first embodiment 100, depicted in FIG. 1A, 1B and 1C except:
- instead of four electrodes comprised in the first surface 310, this embodiment comprises two electrodes in the first surface 310 - nominally an electrode of the first type 200a and nominally an electrode of the second type 400a. From proximal to distal end, the order depicted is 200a, 400a - in other words, an electrode of the first type 200a is proximate an electrode of the second type 400a in the first surface 310.
- the distal end of the stimulator 101 also comprises two electrodes in the second surface 320 - a further electrode nominally of the first type 200b and a further electrode nominally of the second type 400b. From proximal to distal end, the order depicted is 200b, 400b - in other words, an electrode of the first type 200b is proximate an electrode of the second type 400b in the second surface 320.
- In Fig. 2B, the view of the second surface 320 depicts the two electrodes 200a, 400a comprised in that surface, and one or more interconnections 250 are depicted using a dotted line;
- In Fig. 2C, the view of the second surface 320 depicts the two electrodes 200b, 400b comprised in that surface, and one or more interconnections 250 are depicted using a dotted line;

In this embodiment 101, the electrodes of the first type 200a, 200b are nominally configured and arranged to be operated as a stimulation electrode, and the electrodes of the second type 400a, 400b are nominally configured to be operated as a return electrode.

Three main configurations are thus provided:
- a stimulation / return electrode pair 200a / 400a proximate the first surface 310; or
- a stimulation / return electrode pair 200b / 400b proximate the second surface 320; or
- a combination thereof.

This may be advantageous if it is uncertain whether the implantable distal end of the foil-like substrate 300 may be "above" or "below" the targeted tissue such as "above" or "below" a nerve. This may be determined after implantation by attempting stimulation in each nominal configuration and observing and/or measuring the presence of neural stimulation.

As discussed above, in relation to FIG. 1A, 1B and 1C, each electrode 200a, 200b, 400a, 400b may be operated as one or more stimulation electrodes or operated as one or more return electrodes.

In an embodiment, FIG. 3A, 3B and 3C depict longitudinal cross-sections through a third embodiment 102 of an implantable stimulator. It is similar to the second embodiment 101, depicted in FIG. 2A, 2B and 2C except:
- interconnections 250 are disposed at approximately the same disposition along the second transverse axis 750, as depicted in FIG. 3A. The lines 250 are hatched to indicate that they are not be depicted as being in the same longitudinal cross-section - there are interconnections 250 disposed at substantially different positions along the first transverse axis 700;
- interconnections 250 are disposed at substantially different dispositions along the first transverse axis 700, as depicted in FIG. 3B and 3C as two adjacent dashed lines between the electrode array 200, 400 and the pulse generator 500;
- instead of nominally comprising an electrode of the first 200 and second type 400 in the first surface 310, the first surface 310 comprises a first 200a and second 200b electrode nominally of the first type 200;
- instead of nominally comprising an electrode of the first 200 and second type 400 in the second surface 320, the second surface 320 comprises a first 400a and second 400b electrode nominally of the second type 400;

In this embodiment 102, the electrodes of the first type 200a, 200b are nominally configured and arranged to be operated as a stimulation electrode, and the electrodes of the second type 400a, 400b are nominally configured to be operated as a return electrode.

Three main configurations are thus provided:
- a stimulation / return electrode pair 200a / 400a for stimulating between the first surface 310 and second surface 320 proximate the location of this electrode pair; or
- a stimulation / return electrode pair 200b / 400b for stimulating between the first surface 310 and second surface 320 proximate the location of the electrode pair; or
- a combination thereof.

This may be advantageous to correct for a longitudinal misalignment, or to simply allow the healthcare professional to select the most effective stimulation location.

As discussed above, in relation to FIG. 2A, 2B and 2C, each electrode 200a, 200b, 400a, 400b may be operated as one or more stimulation electrodes or operated as one or more return electrodes.

Additionally or alternatively, one or more electrodes of the same type 200a, 200b or 400a, 400b may be electrically connected to each other by suitably configuring the one or more interconnections 250. They will then be operated substantially the same. This may be used to anticipate and/or correct for a misalignment and/or lead migration as longitudinal positioning is less sensitive (a stimulation is provided over a greater longitudinal and or transverse extent).

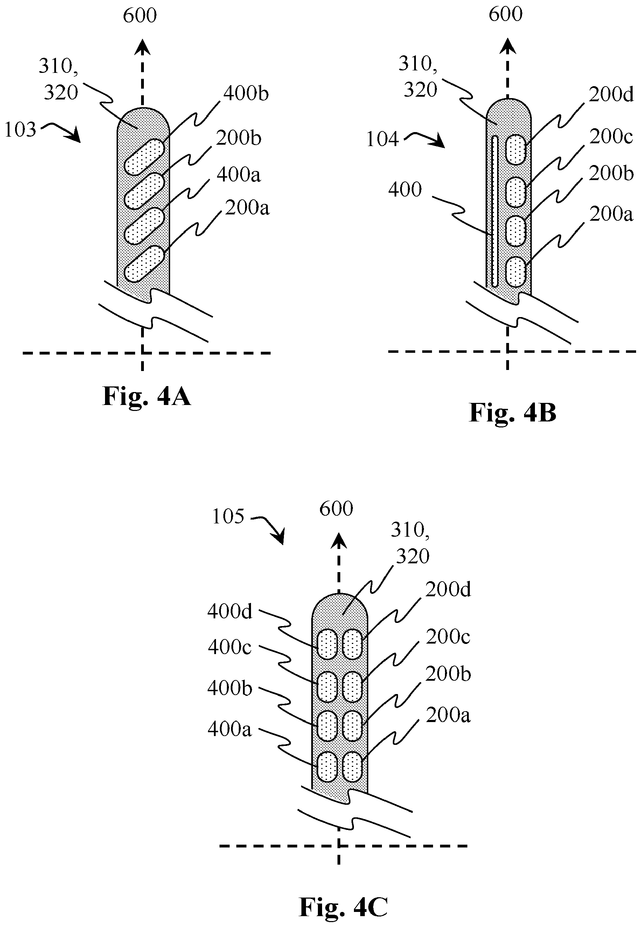

FIG. 4A, 4B and 4C depict alternative electrode array 200, 400 configurations suitable for being comprised in an implantable stimulator 100, 101, 102 as described herein.

FIG. 4A depicts an implantable distal end of a further embodiment 103 of a stimulator. Similar to the distal end depicted in FIG. 1C, the first surface 310 comprises:
- two electrodes 200a, 200b of a first type and two electrodes 400a, 400b of a second type. From proximal to distal end, the order depicted is 200a, 400a, 200b, 400b - in other words, each electrode of the first type 200a, 200b is proximate an electrode of the second type 400a, 400b and comprised in the same surface 310.

The distal end depicted in FIG. 4A is the same as that depicted in FIG. 1A, except:
- the electrodes 200, 400 are extended at angle to the longitudinal axis 600. This may reduce the sensitivity to longitudinal misalignment because the longitudinal locations over which tissue stimulation may be provided are increased.

Additionally or alternatively, the second surface 320 may similarly comprise two electrodes 200a, 200b of the first type and two electrodes 400a, 400b of the second type.

As discussed above, each electrode 200a, 200b, 400a, 400b may be operated as one or more stimulation electrodes or operated as one or more return electrodes.

FIG. 4B depicts an implantable distal end of a further embodiment 104 of a stimulator. Similar to the distal end depicted in FIG. 1C, the first surface 310 comprises four electrodes. However, in this embodiment 104, the first surface 310 comprises:
- four electrodes 200a, 200b, 200c, 200d of a first type and an electrode 400 of a second type. From proximal to distal end, the order depicted is 200a, 200b, 200c, 200d. Transversely adjacent to the four electrodes of the first type 200 is an electrode of the second type 400, extending longitudinally to be adjacent to each electrode of the first type 200.

Nominally, the electrodes of the first type 200 may be operated as one or more stimulation electrodes. The electrode of the second type 400 may be nominally operated as a return electrode for one or more of the stimulation electrodes.

This may reduce the sensitivity to longitudinal misalignment because the four different longitudinal locations are provided which may be selected for stimulation over which tissue stimulation may be provided are increased.

Additionally or alternatively, the second surface 320 may similarly comprise four electrodes 200a, 200b, 200c, 2003 of the first type and one adjacent and longitudinally extended electrode 400 of the second type.

As discussed above, each electrode 200a, 200b, 200c, 200d, 400 may be operated as one or more stimulation electrodes or operated as one or more return electrodes.

FIG. 4C depicts an implantable distal end of a further embodiment 105 of a stimulator. Similar to the distal end depicted in FIG. 4B, the first surface 310 comprises four electrodes 200a, 200b, 200c, 200d of a first type. However, in this embodiment 105, the first surface 310 further comprises four adjacent electrodes 400a, 400b, 400c, 400d of a second type. From proximal to distal end, the order depicted is 200a/400a, 200b/400b, 200c/400c, 200d/400d. Transversely adjacent to each of the four electrodes of the first type 200 is an electrode of the second type 400 at approximately the same disposition along the longitudinal axis 600.

Nominally, the electrodes of the first type 200 may be operated as one or more stimulation electrodes. The electrodes of the second type 400 may be nominally operated as a return electrode for one or more of the stimulation electrodes. Nominally, adjacent electrodes may be considered as a stimulation/return pair 200/400.

In other words, a 2×4 electrode array is provided - two along a transverse axis and four along the longitudinal axis.

This may reduce the sensitivity to longitudinal misalignment because the four different stimulation/return 200/400 pairs are provided at substantially different longitudinal locations are provided which may be selected for stimulation over which tissue stimulation may be provided are increased.

Additionally or alternatively, the second surface 320 may similarly comprise four electrodes 200a, 200b, 200c, 200d of the first type and four adjacent electrodes 400a, 400b, 400c, 400d of the second type.

As discussed above, each electrode 200a, 200b, 200c, 200d, 400a, 400b, 400c, 400d may be operated as one or more stimulation electrodes or operated as one or more return electrodes. This may also reduce the sensitivity to a transverse misalignment.

The stimulator 100, 101, 102, 103, 104, 105 may further comprise:
- an energy receiver, configured and arranged to wirelessly receive energy from an associated energy transmitter when the associated energy transmitter is proximate;
the pulse generator 500 being further configured and arranged to receive electrical energy from the energy receiver for its operation.

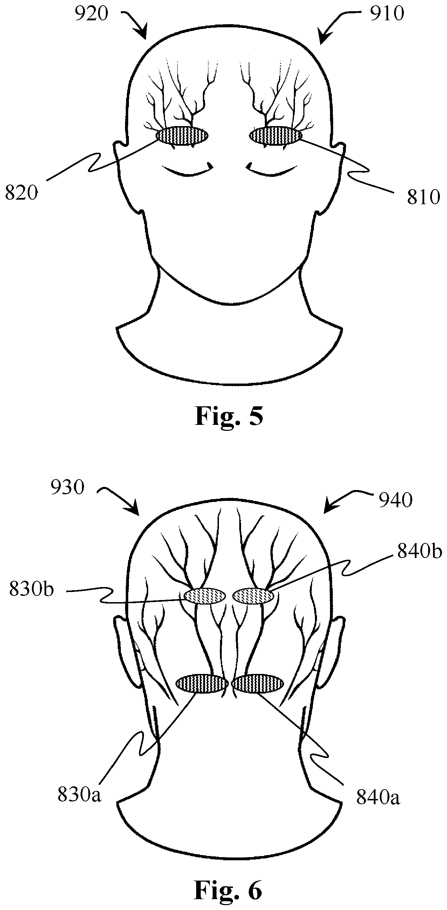

FIG. 5 and FIG. 6 depict configurations of nerves that may be stimulated using a suitably configured implantable distal end of stimulators 100, 101, 102, 103, 104, 105 to provide neurostimulation to treat conditions such as headaches or primary headaches.

FIG. 5 depicts the left supraorbital nerve 910 and right supraorbital nerve 920 which may be electrically stimulated using a suitably configured device. FIG. 6 depicts the left greater occipital nerve 930 and right greater occipital nerve 940 which may also be electrically stimulated using a suitably configured device.

Depending on the size of the region to be stimulated and the dimensions of the part of the device to be implanted, a suitable location is determined to provide the electrical stimulation required for the treatment. Approximate implant locations for the distal part of the stimulation device comprising stimulation devices 100, 101, 102, 103, 104, 105 are depicted as regions:
- location 810 for left supraorbital stimulation and location 820 for right supraorbital stimulation for treating chronic headache such as migraine and cluster.
- location 830a or 830b for left occipital stimulation and location 840a or 840b for right occipital stimulation for treating chronic headache such as migraine, cluster, and occipital neuralgia. Locations 830b, 840b for stimulation are located superior ("above") to the (external) occipital protuberance inion.

In many cases, these will be the approximate locations 810, 820, 830a/b, 840a/b for the implantable stimulator 100, 101, 102, 103, 104, 105.

For each implant location, 810, 820, 830a/b, 840a/b a separate stimulation system may be used. Where implant locations 810, 820, 830a/b, 840a/b are close together, or even overlapping, a single stimulation system may be configured to stimulate at more than one implant location 810, 820, 830a/b, 840a/b.

A plurality of stimulation devices 100, 101, 102, 103, 104, 105 may be operated separately, simultaneously, sequentially or any combination thereof to provide the required treatment.

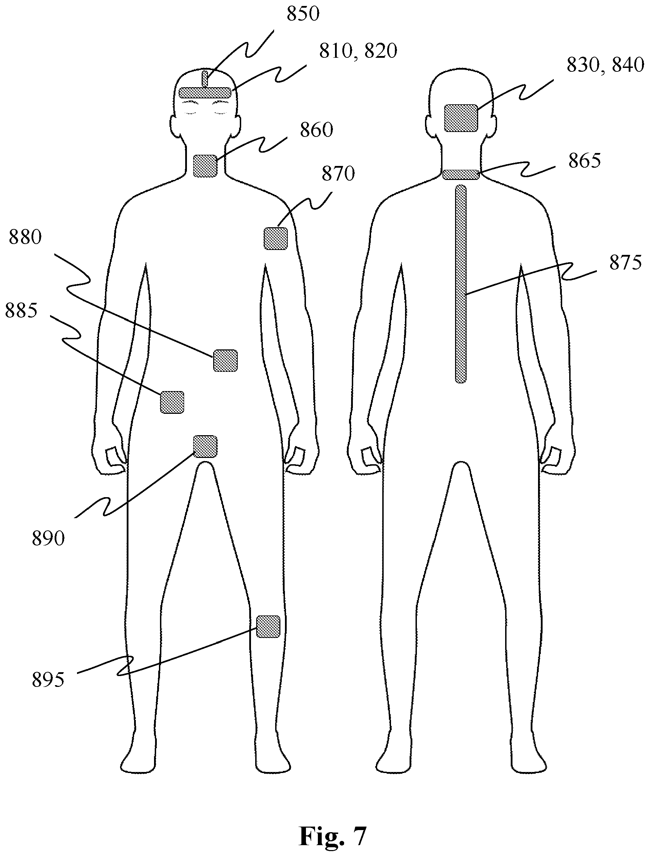

FIG 7 depict further configurations of nerves that may be stimulated using a suitably configured improved implantable stimulator 100, 101, 102, 103, 104, 105 to provide neurostimulation to treat other conditions. The locations depicted in FIG. 5 and FIG. 6 (810, 820, 830, 840) are also depicted in FIG. 7.

Depending on the size of the region to be stimulated and the dimensions of the part of the device to be implanted, a suitable location is determined to provide the electrical stimulation required for the treatment. Approximate implant locations for the part of the stimulation device comprising stimulation electrodes are depicted as regions:
- location 810 for cortical stimulation for treating epilepsy;
- location 850 for deep brain stimulation for tremor control treatment in Parkinson's disease patients; treating dystonia, obesity, essential tremor, depression, epilepsy, obsessive compulsive disorder, Alzheimer's, anxiety, bulimia, tinnitus, traumatic brain injury, Tourette's, sleep disorders, autism, bipolar; and stroke recovery
- location 860 for vagus nerve stimulation for treating epilepsy, depression, anxiety, bulimia, obesity, tinnitus, obsessive compulsive disorder, heart failure, Crohn's disease and rheumatoid arthritis;
- location 860 for carotid artery or carotid sinus stimulation for treating hypertension;
- location 860 for hypoglossal & phrenic nerve stimulation for treating sleep apnea;
- location 865 for cerebral spinal cord stimulation for treating chronic neck pain;
- location 870 for peripheral nerve stimulation for treating limb pain, migraines, extremity pain;
- location 875 for spinal cord stimulation for treating chronic lower back pain, angina, asthma, pain in general;
- location 880 for gastric stimulation for treatment of obesity, bulimia, interstitial cystitis;
- location 885 for sacral & pudendal nerve stimulation for treatment of interstitial cystitis;
- location 885 for sacral nerve stimulation for treatment of urinary incontinence, fecal incontinence;
- location 890 for sacral neuromodulation for bladder control treatment; and
- location 895 for fibular nerve stimulation for treating gait or footdrop.

Other conditions that may be treated include gastro-esophageal reflux disease, an autoimmune disorder, inflammatory bowel disease and inflammatory diseases.

The conformability and reduced thickness of the substrate 100 and electrode array 200, 400 makes one or more implantable stimulators 100, 101, 102, 103, 104, 105 highly advantageous for the stimulation of one or more nerves, one or more muscles, one or more organs, spinal cord tissue, brain tissue, one or more cortical surface regions, one or more sulci, and any combination thereof.

The implantable stimulators 100, 101, 102, 103, 104, 105 described above in relation to FIG. 1 to FIG.4 may be generally described as embodiments configured and arranged for improved conformance.

The stimulator 100, 101, 102, 103, 104, 105 may be further modified. According to the claimed invention the foil-like substrate 300 and pulse generator 500 are embedded in one or more flexible bio-compatible encapsulation layers, such as those described below. These layers comprise a Polydimethylsiloxane (PDMS).

The implantable electrical devices 1100, 1101, 1102 described below in relation to FIG. 8 to FIG. 12 may be generally described as reference-embodiments configured and arranged for improved encapsulation. As described below, they may be comprised in an implantable medical device 1110, 1111 configured and arranged to provide a degree of stimulation.

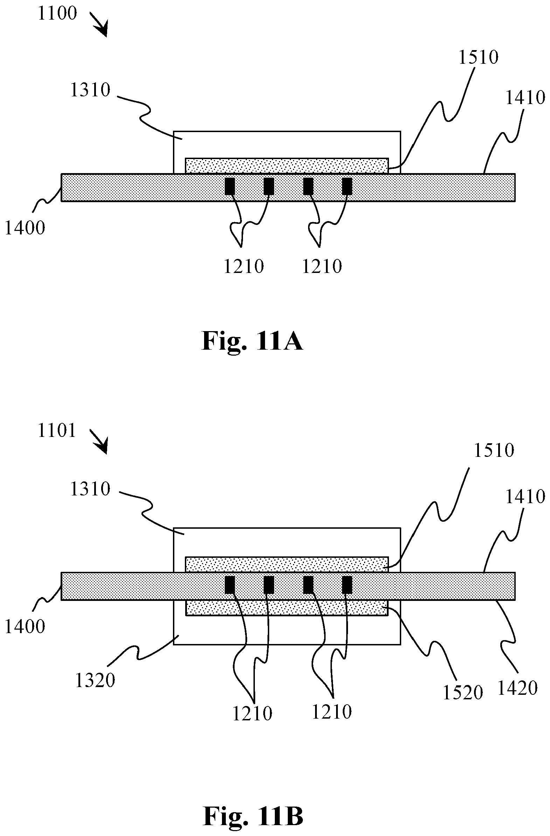

FIG. 11A depicts a cross-section through an improved implantable electrical or electronic device 1100. It comprises:
- a substrate 1400 having a first surface 1410 and one or more electrical conductors 1210.

Optionally, the substrate 1400 may be substantially biocompatible - however, the use of one or more encapsulation layers 1310 may allow substrates 1400 and electrical conductors 1210 which are not biocompatible, partially biocompatible, or significantly biocompatible, to be used.

In general, the degree of biocompatibility of a material or layer may be determined by measuring the degree of tissue reaction and the length of period during which it is considered biostable. A low degree of tissue reaction and/or long period of biostability indicates a high degree of biocompatibility.

The substrate 1400 is further configured and arranged to be substantially flexible - in other words, the substrate is pliant or flexible or compliant (or conformable) to a substantial degree. The degree of flexibility may be adapted using parameters, such as:
- dimensioning of the device 1100 elements, and/or
- inclusion of materials and substances with desired properties, and/or
- combinations of materials and substances used, and/or
- percentages of materials and substances used, and/or
- inclusion of recesses, openings, apertures, reinforcement.

Additionally or alternatively, the skilled person will realize that the degree of flexibility may be adapted using parameters described above for the substrate 300 described in relation to FIG. 1 to FIG. 4.

The one or more electrical conductors 1210 are depicted very schematically - they may be conductors embedded in or deposited onto the substrate 1400 - for example, by having a single polymer layer and applying conductive material using suitable deposition techniques known from the semiconductor industry. The one or more conductors 1210, such as a metal, may be formed as required - for example, in one or more conductive elements: wire, strand, foil, lamina, plate, and/or sheet. Optionally, the one or more conductors may be positioned between the outer surfaces of the substrate 1400;

The device 1100 further comprises:
- a first biocompatible encapsulation layer 1310 comprising a polydimethylsiloxane (PDMS) rubber; and
- a first adhesion layer 1510,

In the context of this disclosure, a ceramic should be considered as an advanced ceramic and/or an industrial ceramic, providing a relatively high degree of thermal stability, wear-resistance and resistance to corrosion.

The most suitable ceramic materials are those with a high degree of adhesion to the encapsulant layer and/or substrate, and capable of being applied in a relatively uniform coating to provide a relatively low degree of permeability to moisture. A ceramic material in this context may be an inorganic, non-metallic or metallic, often crystalline oxide, nitride or carbide material. Some elements, such as carbon or silicon, are also considered ceramics. A non-metallic ceramic may comprise both non-metallic and metallic elements.

Optionally, the first adhesion layer 1510 may be substantially biocompatible - however, the use of one or more encapsulation layers 1310 may allow one or more adhesion layers 1510 which are not biocompatible, partially biocompatible, or significantly biocompatible, to be used.

The first adhesion layer 1510 and the first encapsulation layer 1310 are configured and arranged to resist the ingress of fluids from a human or animal body into at least a portion of the first surface 1410. The configuration and arrangement are further described below.

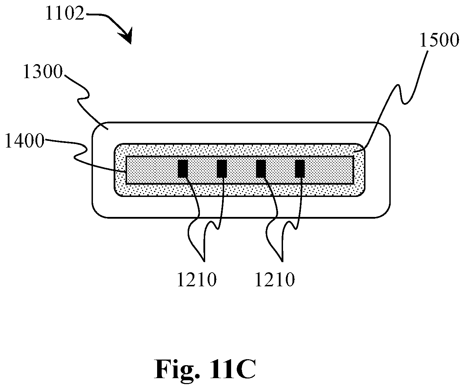

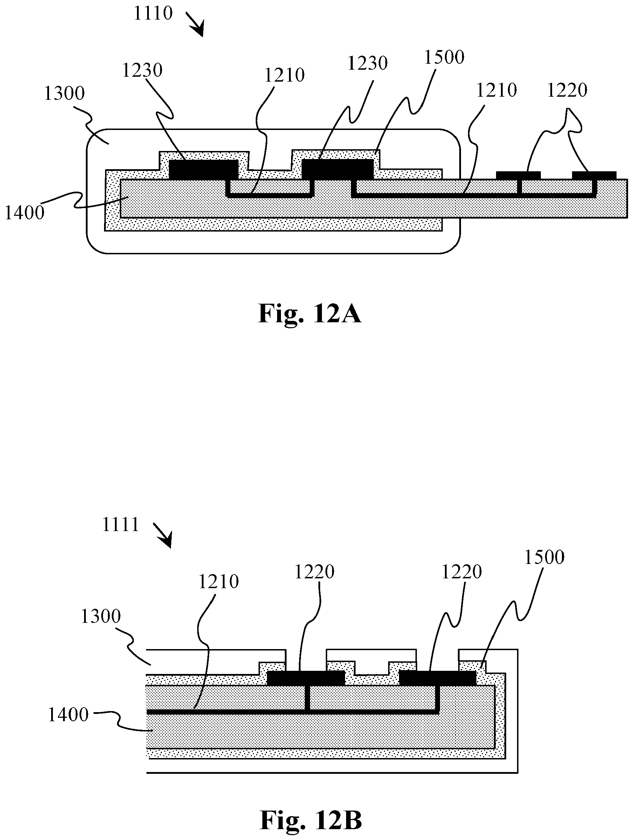

As depicted in FIG. 11A, the extent of the adhesion/encapsulation layer 1510/1310 in this cross-section may be less than the extent of the substrate 1400. In general, the extent of the adhesion/encapsulation layer 1510/1310 may be larger than, equal to or less than the extent of the substrate 1400. A "larger than" embodiment for the adhesion and encapsulation layers is depicted in FIG. 11C. Further "less than" embodiments for the adhesion and encapsulation layers are depicted in FIG. 11B and FIG. 12A.

In general, the portion of the first surface 1410 being protected against ingress of fluids is equal to or less than the extent of the adhesion/encapsulation layer 1510/1310.

As depicted in FIG. 11A, the extent of the adhesion layer 1510 in this cross-section may be less than the extent of the encapsulation layer 1310 - in some configurations, this may be advantageous as the edges of the adhesion layer 1510 are at least partially encapsulated 1310. In general, the extent of the adhesion layer 1510 may be larger than, equal to or less than the extent of the encapsulation layer 1310. Further "less than" embodiments are depicted in FIG. 11C and FIG. 12A. An "equal to" portion of a substrate is depicted in FIG. 12B.

In a preferred embodiment, the extent of the adhesion layer 1510 is equal to or larger than the extent of the encapsulation layer 1310 - this may be advantageous in certain configurations as the surface area of encapsulant 1310 in direct contact with the surface 1410 of the substrate 1400 is greatly reduced. In some cases, this surface area may be substantially zero, further reducing the possibility of fluid ingress. A "substantially zero" embodiment is depicted in FIG. 11C and a portion of a substrate depicted in FIG. 12B.

FIG. 11B depicts another implantable electrical or electronic device 1101. It is the same as the implantable electrical device 1100 depicted in FIG. 11A, except for further comprising:
- a second surface 1420;
- a second biocompatible encapsulation layer 1320, comprising a polydimethylsiloxane (PDMS) rubber; and
- a second adhesion layer 1520, comprising a ceramic material, disposed between the second planar surface 1420 and the second encapsulation layer 1320. The second adhesion layer 1520 is further configured and arranged to conform to the second surface 1420 - in other words, it is a conformal layer.

The second adhesion layer 1520 and the second encapsulation layer 1320 are configured and arranged to resist the ingress of fluids from a human or animal body into at least a portion of the second surface 1420. The configuration and arrangement are further described below.

The second encapsulation layer 1320 may be substantially identical, similar to a high degree or substantially different to the first encapsulation layer 1310.

The second adhesion layer 1520 may be substantially identical, similar to a high degree or substantially different to the first adhesion layer 1510.

Although the first surface 1410 and second surface 1420 are depicted as opposite faces of a substrate in FIG. 11B, other combinations are possible, such as:
- applying the first adhesion/encapsulation layer 1310/1510 and the second adhesion/encapsulation layer 1320/1520 to different regions of the first surface 1410;
- applying the first adhesion/encapsulation layer 1310/1510 and the second adhesion/encapsulation layer 1320/1520 to different regions of the second surface 1420;
- the first surface 1410 and second surface 1420 being adjacent to each other;
- the first surface 1410 and second surface 1420 being opposite to each other;
- the first surface 1410 and second surface 1420 being at a predetermined angle to each other;
- the first surface 1410 and second surface 1420 being substantially perpendicular to each other.

FIG. 11C depicts a further implantable electrical or electronic device 1102. It is the same as the implantable electrical device 1100 depicted in FIG. 11A, except in this cross-section:
- the substrate 1400 comprises four protected surfaces, each surface being protected by a further adhesion layer 1500 and a further encapsulation layer 1300;
- the extent of the further adhesion layer 1500 is larger than the extent of substrate 1400 for each protected surface;
- the extent of the further encapsulation layer 1300 is larger than the extent of the substrate 1400 for each protected surface; and
- the extent of the further adhesion layer 1500 is less than the extent of the encapsulation layer 1300 for each protected surface.

Functionally, it may also be considered that the further encapsulation layer 1300 comprises the first 1310 and second 1320 encapsulation layers depicted in FIG. 11B.

Functionally, it may also be considered that the further adhesion layer 500 comprises the first 1510 and second 1520 adhesion layers depicted in FIG. 11B.

Functionally, it may also be considered that the substrate 1400 depicted in FIG. 11C comprises the protected portions of the first 1410 and second 1420 surfaces depicted in FIG. 11B. However, the substrate 1400 depicted in FIG. 11C, comprises two or more further protected surfaces, adjacent to such a protected first or second surface.

The further encapsulation layer 1300 of FIG. 11C may be substantially identical, similar to a high degree or substantially different to the first encapsulation layer 1310 depicted in FIG. 11A or 11B. The further encapsulation layer 1300 of FIG. 11C may be substantially identical, similar to a high degree or substantially different to the second encapsulation layer 1320 depicted in FIG. 11B.

The further adhesion layer 1500 of FIG. 11C may be substantially identical, similar to a high degree or substantially different to the first adhesion layer 1510 depicted in FIG. 11A or 11B. The further adhesion layer 1500 of FIG. 11C may be substantially identical, similar to a high degree or substantially different to the second adhesion layer 1520 depicted in FIG. 11B.

The further embodiment 1102 may be advantageous because:
- the portion of the surfaces of the substrate 1400 being protected against ingress of fluids is less than the extent of the further encapsulation layer 1300;
- the edges of the further adhesion layer 1500 are substantially encapsulated 1300; and
- the surface area of encapsulant 1300 in direct contact with a surface of the substrate 1400 is close to or substantially zero.

Experiments were performed to establish the suitability of a specific adhesion layer 1510, 1520 to provide a high degree of bonding to a PDMS.

### A. Sample Preparation

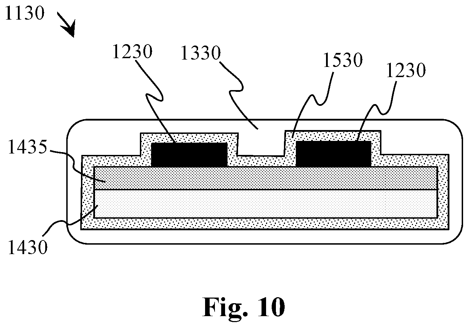

### FIG. 10 depicts a cross-section through the test sample 1130

### 1) IDC using Pt Metallization

Interdigitated capacitors (IDC) 1230 were used to evaluate encapsulation performance - approximately 600nm of Pt (Platinum) was sputtered on top of a 1 µm (1 micron) thick plasma enhanced chemical vapor deposition (CVD) SiO2 layer 1435 with an intermediate 10nm titanium adhesion layer. More details on these IDC 1230 are found in "Silicone rubber encapsulation for an endoscopically implantable gastrostimulator", Lonys, Vanhoestenberghe, Julemont, Godet, Delplancke, Mathys and Nonclercq, Med. Biol. Eng. Comput. 53 319-29, 2015. The SiO2 layer 1435 was provided on a silicon substrate 1430.

### 2) ALD Coating

Atomic layer deposition (ALD) is a coating process that may be used to create nm-thick conformal coatings. The ALD coating was applied using the PICOSUN^{®} R-200 Advanced ALD reactor under reduced pressure (N2 atmosphere) of about 1 mbar (1hPa).

The R-200 Advanced, from Picosun Oy, Finland, provides very high quality ALD film depositions. It is suggested by the manufacturer as suitable for depositions including: Al2O3, TiO2, SiO2, Ta2O5, HfO2, ZnO, ZrO2, AlN, TiN, metals such as Pt or Ir.

It comprises a remote microwave plasma generator, with adjustable 300 - 3000 W power, 2.45 GHz frequency, mounted to the loading chamber and connected to the reaction chamber. Up to twelve sources with six separate inlets may be used - seven if the plasma option is chosen. The precursor sources may comprise liquid, gaseous and/or solid chemicals. Precursors may also include ozone and/or plasma. The remote plasma option allows deposition of metals with a greatly reduced risk of short-circuiting and/or plasma damage. The processing temperature may in general be 50 - 500°C. Plasma may generally be used up to approximately 450°C, or up to approximately 650 °C with a heated sample holder.

It comprises a hot-wall and substantially separate inlets and instrumentation providing a relatively low particle (or substantially particle-free) processing adaptable on a wide range of materials on wafers, 3D objects, and nanoscale features. It provides a high degree of uniformity, even on porous, through-porous, high aspect ratio (up to 1:2500), and nanoparticle samples using their proprietary Picoflow^{™} diffusion enhancer. This enhancer provides a protective gas flow in an intermediate space to greatly reduce back-diffusion of the plasma species.

A suitable ALD process, for forming a monolayer comprising a first and second element, may comprise:
- loading a substrate as a sample into a reaction space;
- introducing a quantity of first molecules comprising the first element into the reaction space whereby at least a first portion of the first molecules adsorb to a surface of the substrate; and
- introducing a quantity of second molecules comprising the second element into the reaction space whereby at least a second portion of the second molecules reacts with the first portion on the surface of the substrate to form a monolayer of a compound comprising the first and second element.

Using the Picohot^{™} source system (PH-300) and PicoSolution options for the R-200 Advanced, precursors were vaporized from stainless-steel precursor bottles at increased temperature and at room temperature. The Picohot^{™} 300 source system allows source heating up to 300 degr. C, and is suggested by the manufacturer to be suitable with source chemicals having a vapor pressure of at least 2 mbar at source temperature. The Picosolution^{™} 600 source system allows liquid precursors to be used, and are suggested by the manufacturer to be suitable with source chemicals having a vapor pressure of at least 10 mbar at source temperature.

Thermal ALD-processes at 200 degr. C were applied with layer-by-layer deposition method where the two different precursor materials (separated by N2 purge to remove surplus molecules from the reaction space) were used to build up a HfO2 (hafnium dioxide) coating 1530 - this is depicted in FIG. 10 as a coating 1530 substantially covering the external surfaces of the substrate 1430, 1435 and the IDC sensors 1230.

An optional stabilization time of approximately 90 minutes was used at 200 degr. C. Ten layers of approximately 5nm were applied to provide an ALD layer of approximately 50nm.

It is believed that ALD may be advantageous to create an ultra-thin conformal coating with low defects and/or reduced pinhole formation. Also, the deposition temperature for ALD may be kept below 200°C which is advantageous for devices incorporating sensitive metallization and/or polymers.

### 3) PDMS encapsulation

Samples were encapsulated with a layer comprising a substantially biocompatible PDMS (MED2-6215, NuSil Carpinteria, USA) 1330.

### From nusil.com/product/med-6215_optically-clear-low-consistency-silicone-elastomer:

MED-6215 is an optically clear, low consistency silicone elastomer. It is provided as two-parts which are solvent free and have a relatively low viscosity. It cures with heat via addition-cure chemistry. The mix ratio is 10:1 (Part A: Part B).

MED-6215 is considered substantially biocompatible - the manufacturer suggests that it may be used in human implantation for a period of greater than 29 days.

### Uncured:

| **Typical properties** | **Average Result** | **Standard** | **Nusil Test (NT-TM)** |
|---|---|---|---|
| Appearance | Translucent | ASTM D2090 | 002 |
| Viscosity, Part A | 5,500 cP (5,500 mPas) | ASTM D1084, D2196 | 001 |
| Viscosity, Part B | 95 cP (95 mPas) | ASTM D1084 D2196 | 001 |
| Work Time | 5 hours | ------ | 008 |

### Cured: 15 minutes at 150°C (302°F)

| **Typical properties** | **Average Result** | **Standard** | **Nusil Test (NT-TM)** |
|---|---|---|---|
| Specific Gravity | 1.03 | ASTM D792 | 003 |
| Durometer, Type A | 50 | ASTM D2240 | 006 |
| Tensile Strength | 1,250 psi (8.6 MPa) | ASTM D412 | 007 |
| Elongation | 100% | ASTM D412 | 007 |
| Tissue Culture (Cytotoxicity Testing) | Pass | USP ISO 10993-5 | 061 |
| Elemental Analysis of Trace Metals | Pass | ASTM E305 | 131 |

| **Property** | **Average Result** |
|---|---|
| Durometer | 50 Type A |
| Viscosity | 3,800 MPa*s (3800 cP) |
| Work Time | 5 hours |
| Tensile | 8.62 MPa (1250 psi) |
| Appearance | Transparent |
| Cure | 15 minutes / 150 °C |
| Cure System | Platinum |
| Elongation | 100% |
| Mix Ratio | 10:1 |
| Specific Gravity | 1.03 |
| Tack Free Time | 16 hours |
| Comment | Clear, 1.41 R.I. |

The manufacturer suggests silicone primer Nu-Sil MED 1-161 as a primer to further improve adhesion of MED-6215 to various substrates including: metals (such as stainless steel, steel, copper and aluminum), ceramic materials, rigid plastics, and other silicone materials.

MED-6215 is available in medical grade - in other words, substantially biocompatible and suitable for use in a medical implantable device. This is realized by ensuring all raw materials, intermediates, and finished products (for Medical Grade) are manufactured with applicable GMP and/or appropriate regulatory standards: cGMP 21 CFR § 820 (Device), cGMP 21 CFR § 210-211(Drug/API) and ISO 9001.

A dip-coating process was used for the encapsulation. The average relatively low viscosity, for example, 4000 to 7000 cP (mPas), appears to have allowed the PDMS to more easily flow over the sample. The thickness of the PDMS 1330 was estimated to be between 50 and 200 µm (micron).

### B. Experimental Set-up

The lifetime reliability of ALD coatings may depend on factors such as the conformality and adhesion of the layer, and its stability in ionic media. This was measured using the IDC's impedance after an extended soak test.

Extended soaking used phosphate buffered saline (PB S) at approximately room temperature (approx. 23°C).

Electrochemical impedance spectrometry (EIS) was carried out to evaluate the performance of the ALD and ALD-PDMS coatings using the methods described in "Apparatus to investigate the insulation impedance and accelerated life-testing of neural interfaces", Donaldson, Lamont, Shah Idil, Mentink, Perkins, J. Neural Eng, 2018, 10.1088/1741 -2552/aadeac.

Measurements used a Solartron Modulab with a potentiostat in combination with a frequency response analyzer. Measurements were performed in a two-cell electrode configuration between the combs of the IDC structure. A Faraday cage was also used.

### III. RESULTS AND DISCUSSION

### A. Measurement Results

After sample preparation and submersion in saline, EIS measurements were performed.

FIG. 8A and 8B show the EIS results 1700, 1710 for three samples.

FIG. 8A depicts Bode plots 1700, with impedance magnitude along the vertical (Y) axis from 10¹ to 10¹¹ |Z| Ohm, and frequency along the horizontal (X) axis from 10⁻² to 10⁵ Hz:
a bare IDC with exposed Pt metal 1701, forming an approximately straight line from approx. 10⁻², 5×10⁶ to 10⁴, 10², followed by a further straight line to 10⁵, 10²;
- an IDC coated with HfO2 ALD 1702, forming an approximately straight line from 10⁻², 10⁹ to 10⁵, 10³ and
- an IDC coated with an ALD-PDMS bilayer 1703, forming an approximately straight line from 10⁻², 10¹¹ to 10⁵, 10⁵.

FIG. 8B depicts Bode plots 1710, with phase along the vertical (Y) axis from 0 to -90 degrees, and frequency along the horizontal (X) axis from 10⁻² to 10⁵ Hz:
- a bare IDC with exposed Pt metal 1711, forming a curve passing through 10⁻², - 20 to 10⁰, -70 to 10², -80 to 10⁻⁴, -20 to 10⁻⁵, 0;
- an IDC coated with HfO2 ALD 1712, forming a curve passing through 10⁻², -60 to 10⁰, -80 to 10², -90 to 10⁻⁴, -80 to 10⁻⁵, -70; and
- an IDC coated with an ALD-PDMS bilayer 1713, forming a curve passing through 10⁻², -80 to 10⁰, -90 to 10², -90 to 10⁻⁴, -80 to 10⁻⁵, -90.

For the bare IDC 1701, 1711, in the middle frequency band (10⁰Hz - 10³Hz), the phase 1711 appears to be relatively constant at approximately -80 degr. At lower frequencies (approx. 10⁻² Hz), the polarization resistance appears to be dominant, resulting in a phase of approximately -20 degr. It is believed that this indicates the metal fully exposed to an electrolyte.

The ALD-coated IDC 1702, 1712, appeared to show relatively higher impedance values - this suggests a more capacitive behavior across the frequency range. This capacitance is believed to be caused by the Pt metal and electrolyte being separated by an ALD layer, which acts as a dielectric. It is believed that a fully conformal coating on the metal, or high resistance to fluid ingress, would result in a substantially capacitive behavior in the EIS results 1700, 1710.

For the ALD - PDMS bilayer 1703, 1713, the impedance 1703 and phase 1713 results show a substantially capacitive behavior across substantially the whole frequency range, with phase results 1713 close to approximately -90°.

It is believed that any delamination or cracking of the ALD layer may expose more metal to the electrolyte, possibly resulting in a substantially lower impedance and phase angle that is more significantly seen in the lower frequency regions <10⁻¹ Hz. In FIG. 8A and 8B, a comparison between the ALD 1702, 1712 and ALD-PDMS bilayer 1703, 1713 shows an approximately two orders of magnitude higher impedance value 1703 for the bilayer encapsulated IDC 1703, 1713. Furthermore, the phase results 1713 show a substantially more capacitive behavior.

Additionally, metal areas exposed due to ALD defects are also encapsulated with the PDMS, with a specific resistance of approximately 10¹⁵ Ohm.cm. It is believed that any significant delamination of the PDMS from ALD would allow water condensation, resulting in one or more conductive paths between the combs. This may result in a lower impedance and phase angle more significantly seen in the lower frequency regions of approximately <10⁻¹ Hz.

To track changes in the encapsulation and adhesion performance, monthly EIS measurements were done on all samples. The impedance and phase angle at approximately 10⁻² Hz were selected as the reference value to monitor over time.

FIG. 8C and 8D show the adhesion evaluation results 1720, 1730 for two ALD samples and two ALD-PDMS samples over the four hundred and fifty days of soaking. The results depicted in FIG. 8A and 8B were considered as the values measured at T=0 days.

FIG. 8C depicts adhesion evaluation 1720, with impedance magnitude along the vertical (Y) axis from 0 to 10¹¹ |Z| Ohm, and Time along the horizontal (X) axis from 0 to 16 months:
- two IDC's coated with HfO2 ALD 1722a, 1722b, forming an approximately straight line from 0, 10⁹ to 16, 10⁹. Both samples provided substantially the same results, resulting in lines that are substantially overlapping, except for minimal deviations at 0 to 1 months, and 15 to 16 months; and
- two IDC's coated with an ALD-PDMS bilayer 1723a, 1723b, forming an approximately straight line from 0, 10¹¹ to 16, 10¹¹. Both samples provided substantially the same results, resulting in lines that are substantially overlapping.

FIG. 8D depicts adhesive evaluation 1730, with phase along the vertical (Y) axis from -30 to -90 degrees, and Time along the horizontal (X) axis from 0 to 16 months:
- a first IDC 1732a coated with HfO2 ALD, forming an approximately straight line from 0, -70 to 16, -65;
- a second IDC 1732b coated with HfO2 ALD, forming an approximately straight line from 0, -75 to 2, -63 to 4, -65 to 16, 60;
- a first IDC 1733a coated with an ALD-PDMS bilayer, forming an approximately straight line from 0, -83 to 2, -78 to 6, -80 to 16, -80; and
- a second IDC 1733b coated with an ALD-PDMS bilayer, forming an approximately straight line from 0, -80 to 2, -78 to 6, -77 to 10, -80 to 16, -78.

For the ALD-only samples 1722, 1732, a drop in the phase angle 1732a, 1732b was measured after the first month of soaking, suggesting that fluid came into contact with the metals through one or more defects in the ALD layer. Substantially stable results were observed during the extended soaking. This is believed to indicate a substantially high stability of the HfO2 adhesion layer in ionic media and a substantially high degree of adhesion of HfO2 to Pt over an extended period of time. Significant deterioration of the HfO2 layer would be expected to show a relatively higher capacitive behavior, such as a significant drop in the impedance magnitude 1720 - this was not observed. Additionally, any significant delamination of the ALD layer from Pt would be expected to result in a substantially more resistive behavior, originating from the metal being exposed to saline - this was also not observed.

Optical inspections of the ALD samples 1722, 1732 supported these conclusions as no significant layer discoloration or degradation were observed.

For the ALD-PDMS bilayer samples 1723, 1733, substantially stable results were thus recorded over an extended period, suggesting a relatively high degree of adhesion between the two layers, and a substantially higher resistance to the ingress of fluids.

### B. Conclusions

Pt is widely used as for conductors and/or electrode regions due to its high degree of biocompatibility and stability. However, long term stability may be reduced in conventional systems due to the relatively weak adhesion of encapsulants, such as PDMS, parylene and epoxy to Pt.

From the results, it is believed that adding an adhesion layer comprising one or more ceramic materials may be advantageous. In particular, an HfO2 ALD layer with an average thickness of approximately 25nm to 100nm, preferably approximately 50nm, may provide a substantially stable intermediate adhesion layer between Pt and the PDMS. Additionally, a relatively high degree of adherence was also measured between the HfO2 layer and the SiO2 substrate - in particular between the Pt forks.

Where appropriate, a substrate comprising other materials may thus be provided with a layer of SiO2 and/or Pt to improve adhesion to the HfO2 ALD layer.

The ALD-PDMS bilayer of an encapsulation layer 1330 and adhesion layer 1530 appears particularly advantageous:
- the HfO2 ALD adhesion layer appeared to show a significantly higher stability in ionic media, thereby, providing relatively longer resistance against delamination or water permeation through the PDMS encapsulation.
- a PDMS having a relatively low average viscosity , for example 4000 to 7000 cP (mPas), for a significant time period during encapsulation, may further contribute to longer-lasting adhesion and defect reduction due to flowing of PDMS in-between any defects and openings in the ALD layer
- PDMS-type materials are, in general, highly suitable for implantation due to their relatively high degree of biocompatibility. By appropriate selection and processing, many PDMS-type materials may be configured and arranged to be substantially biocompatible.

Polymeric materials comprised in the substrate 1400 are preferably selected for suitability to be flexible, and to comprise the one or more electrical conductors 1210. Preferably, the polymeric substrate materials have a high degree of biocompatibility and durability. Suitable polymer materials for being comprised in substrate 1400 include those mentioned above for conformable substrates in relation to FIG. 1 to FIG. 4. In particular, a polyimide, Parylene C, SU-8, an LCP, a polyurethane, or any combination thereof may be used.

Preferably, the first and/or second surface 1410, 1420 comprise a significant amount of one or more Liquid Crystal Polymers (LCP's). Optionally, the first and/or second surface 1410, 1420 may substantially consist of one or more LCP's. Optionally, the first and/or second surface 1410, 1420 may essentially consist of one or more LCP's.

The table below compares several physical and chemical properties of a typical polyimide and a typical LCP.

| | **Unit** | **LCP Flex** | **Polyimide Flex** |
|---|---|---|---|
| Thickness | Um (micron) | 25, 50, 100 | 12, 25, 50 |
| Dielectric constant (10 GHz) | -- | 2.9 | 3.2 |
| Dissipation factor (10 GHz) | -- | 0.002 | 0.002 |
| Surface resistivity | ohm | 1.0 E16 | 4.0 E13 |
| Volume resistivity | Ohm cm | 1.0 E18 | 2.6 E14 |
| Dielectric strength | kV / mil | 3.5 | 7 |
| Young's modulus | GPa | 2.3 | 7.1 |
| Tensile strength | MPa | 280 | 220 |
| CTE, x-y | ppm/K | 18 | 20 |
| CTE, z | ppm/K | 200 | 120 |
| Solder float temperature | °C | > 288 | > 300 |
| Melting temperature / glass transition | °C | 330 | 343 |
| Moisture absorption (23°C, 24h) | % | 0.04 | 1 |
| Flammability | -- | UL 94 VTM-0 | UL 94 V0 |

Advantageously, the substrate 1400, for example comprising an LCP, has a Young's modulus in the range 2500 to 3600 MPa (2.5 to 3.6 GPa).

Optionally, the substrate 1400 may further comprise one or more electrical or electronic components configured to receive energy when electrical energy is applied to the one or more electrical conductors 1210. For example, they may be inductively-coupled, capacitively-coupled or directly connected. This is particularly advantageous with substrates comprising significant amounts of one or more LCP's as PCB-techniques may be used. Preferably, a bio-compatible metal such as gold or platinum is used.

Preferably, one or more encapsulation layers 1310, 1320 and one or more adhesion layers 1510, 1520 are configured and arranged to resist the ingress of fluids to at least a portion of one or more surfaces 1410, 1420 proximate the one or more components.

For example, the one or more components may be an active component, a passive component, an electronic component, an integrated circuit (IC), an application-specific integrated circuit (ASIC), a field-programmable gate array (FPGA), an analog component, a digital component, a surface-mount device (SMD), a through-hole package, a chip carrier, a pin grid array, a fat package, a small outline package, a chip scale package, a ball grid array, a small-pin-count package, a flexible silicon device, a thin-film transistor (TFT), and any combination thereof.

The one or more electrical components may be configured and arranged to: resist, store charge, induct, sense, stimulate, amplify, process data, detect, measure, compare, switch, time, store data, count, oscillate, perform logic, add, generate stimulation pulses, and any combination thereof.

The substrates 1400 may be further configured and arranged to have a degree of conformance as described above. According to the claimed invention, they are foil-like (or film-like) and follow the contours of underlying anatomical features very closely by being flexible. Very thin foil-like substrates 1400 have the additional advantage that they have increased flexibility.

An implantable electrical device 1100, 1101 as described herein may be comprised in an implantable medical device 1110, 1111. For example, such a medical device 110, 1111 may be configured and arranged to provide a degree of sensing, stimulation, data processing, detection or measurement, data storage, oscillation, logic performance, stimulation pulses generation, or any combination thereof.

The embodiments described above in relation to FIG. 1 to FIG. 4, and in particular the implantable stimulators 101, 102, 103, 104, 105 may comprise an implantable electrical device 1100, 1101, 1102.

As depicted in FIG. 12A, an improved implantable medical device 1110 may be provided by modifying the implantable device 1100, depicted in FIG. 11A. It is the same as the implantable electrical device 1100 depicted in FIG. 11A, except in this cross-section:
- the substrate 1400 comprises three protected surfaces, each surface being protected by a further adhesion layer 1500 and a further encapsulation layer 1300. The three protected surfaces comprise two opposite protected surfaces and a further adjacent surface;
- the extent of the further adhesion layer 1500 is less than the extent of the substrate 1400 for the two opposite protected surfaces. The extent of the further adhesion layer 1500 is greater than the extent of the substrate 1400 for the third adjacent protected surface;
- the extent of the further encapsulation layer 1300 is less than the extent of the substrate 1400 for the two opposite protected surfaces. The extent of the further encapsulation layer 1300 is greater than the extent of the substrate 1400 for the third adjacent protected surface; and
- the extent of the further adhesion layer 1500 is less than the extent of the further encapsulation layer 1300 for each protected surface.

Functionally, it may also be considered that the further encapsulation layer 1300 comprises the first 1310 and second 1320 encapsulation layers depicted in FIG. 11B.

Functionally, it may also be considered that the further adhesion layer 1500 comprises the first 1510 and second 1520 adhesion layers depicted in FIG. 11B.

However, the substrate 1400 depicted in FIG. 12A, comprises a further protected surface, adjacent to such a protected first or second surface.

The further encapsulation layer 1300 of FIG. 12A may be substantially identical, similar to a high degree or substantially different to the first encapsulation layer 1310 depicted in FIG. 11A or 11B. The further encapsulation layer 1300 of FIG. 12A may be substantially identical, similar to a high degree or substantially different to the second encapsulation layer 1320 depicted in FIG. 11B.

The further adhesion layer 1500 of FIG. 12A may be substantially identical, similar to a high degree or substantially different to the first adhesion layer 1510 depicted in FIG. 11A or 11B. The further adhesion layer 1500 of FIG. 12A may be substantially identical, similar to a high degree or substantially different to the second adhesion layer 1520 depicted in FIG. 11B.

The medical device 1110 further comprises:
- one or more stimulation electrodes 1220, configured and arranged to transmit energy to human or animal tissue when electrical energy is applied to the one or more electrical conductors 1210. For example, they may be inductively-coupled, capacitively-coupled or directly connected. In the example depicted, the one or more stimulation electrodes 1220 are directly connected to the one or more electrical conductors 1210. In many neurostimulation applications, a plurality of electrodes 1220 may be required. These may be identical, similar or different to the electrodes 200, 400 described above in relation to FIG. 1 to FIG. 4.

Optionally or additionally, one or more sensors 1230 may similarly be provided - such sensors 1230 are configured to be provided electrical signals and/or data to the one or more electrical conductors 1210. For example, they may be inductively-coupled, capacitively-coupled or directly connected. If a multilayer substrate with electrical interconnections is provided, a high degree of customization is possible. For example, allowing direct measurements of parameters relevant for operation, such as humidity, temperature, electrical resistance and electrical activity.

Typically with neural-stimulation electrodes, one or more electrodes 1220 are configured and arranged to operate as a ground or return electrode - this may be one of the existing electrodes or one or more further electrodes as described above for the first 200a, 200b and second 400a, 400b electrodes described above in relation to FIG. 1 to FIG. 4.

The skilled person will realize that such a stimulation electrode 1220 and/or a tissue sensor is preferably not completely covered by an encapsulation layer 1300 and/or an adhesion layer 1500 as a sufficiently high degree of electrical connection or exposure to the implant environment are required for their function. For example, at least part of a stimulation electrode 1220 and/or tissue sensor is masked during the encapsulation process to provide a conductive surface towards tissue. Additionally or alternatively, portions of the device may not be encapsulated.

FIG. 12A depicts a device 1110 where substantially all of a stimulation electrode 1220 is substantially not covered. In addition, in this cross-section, a portion of the substrate 1400 is substantially not covered, providing a device 1110 with a substantially encapsulated portion and a substantially unencapsulated portion with one or more electrodes 1220. The extent of the further adhesion layer 1500 in this cross-section for the two opposite surfaces is less than the extent of the further encapsulation layer 1300 for these surfaces - this may be advantageous as the edges of the further adhesion layer 1500 are at least partially encapsulated 1300.

Applying this encapsulation to the implantable stimulators described above in relation to FIG. 1 and FIG. 4 generally provides a substantially unencapsulated portion with one or more electrodes 200, 400, and a substantially encapsulated portion comprising a pulse generator 500.

FIG. 12B depicts a further embodiment of a medical device 1111. More particularly, it depicts a cross-section through a portion of the substrate 1400 comprising one or more electrode 1220. The further medical device 1111 is the same as the device 1110 depicted in FIG. 12A except, in general, in this cross-section:
- the substrate 1400 comprises four protected surfaces, each surface being protected by a further adhesion layer 1500 and a further encapsulation layer 1300;
- the extent of the further adhesion layer 1500 is larger than the extent of substrate 1400 for each protected surface;
- the extent of the further encapsulation layer 1300 is larger than the extent of the substrate 1400 for each protected surface; and
- the extent of the further adhesion layer 1500 is less than the extent of the encapsulation layer 1300 for each protected surface.

In this cross-section, "a part" of the one or more stimulation electrodes 1220 is not completely covered to allow electrical connection or exposure to the implant environment after implantation. So, in the regions close to the stimulation electrodes 1220, the general statements made above do not all apply completely. In particular, in this cross-section:
- the further adhesion layer 1500 has been applied to the surface of the substrate 1400 adjacent to the stimulation electrodes 1220 and also applied to edge portions of the surface of the electrodes 1220. This may provide additional protection against ingress at any interface between the electrode 1220 and the substrate 1400; and
- the further encapsulation layer 1300 has been applied to the surface of the substrate 1400 adjacent to the stimulation electrodes 1220. However, is not significantly applied to edge portions of the surface of the electrodes 1220.

In other words, in this cross-section at the edge portions of the surface of the electrodes 1220, the extent of the further adhesion layer 1500 is approximately the same as the extent of the further encapsulation layer 1300.

This may be advantageous in certain configurations as the surface area of further encapsulation layer 1300 in direct contact with the surface of the electrodes 1220 is greatly reduced. In some cases, this surface area may be substantially zero.

Applying this encapsulation to the implantable stimulators described above in relation to FIG. 1 and FIG. 4 generally provides a substantially encapsulated portion in which "a part" of the one or more electrodes 200, 400, and a substantially encapsulated portion comprising a pulse generator 500.

Optionally, it may be advantageous if the extent of the further encapsulation layer 1300 in this cross-section at an edge portion of one or more electrodes 1220 is greater than the extent of the further adhesion layer 1500 - in some configurations, this may be advantageous as the edges of the further adhesion layer 1500 are at least partially encapsulated 1300.

So, the one or more stimulation electrodes 1220 and/or sensor are preferably comprised in a surface, configured and arranged to provide a tissue interface.

As described above, "comprised in a surface" means that the electrodes 1220 are relatively thin (for example, when the substrate conforms to a substantially planar surface, having an extent along a transverse axis. approximately perpendicular to a longitudinal axis of the substrate, of 20 to 50 microns or less. Thinner electrodes may be also be used to further increase the degree of conformability, for example 1 micron or less), and attached to (or at least partially embedded in) the surface.

This is particularly advantageous with substrates comprising significant amounts of one or more LCP's as PCB/metallization-techniques may be used to provide conductive regions, which may be configured and arranged to be electrodes 1220 and/or sensors 1230. As described above, a conductive material is preferably used such as gold, platinum, platinum black, TiN, IrO₂, iridium, and/or platinum/iridium alloys and/or oxides. Conductive polymers, such as Pedot, may also be used. Preferably, bio-compatible conductive materials are used.

As described above, thicker metal layers are generally preferred over thinner metal layers for electrodes 1220 because they can be subjected to bodily substances that may dissolve the metal. However, thicker metal layers typically increase rigidity (reduce conformability) proximate the thicker layer.

In a second set of experiments, adhesion of PDMS MED2-4213 from NuSil to an LCP substrate was investigated using two different substrates and two different PDMS casting processes.

Different methods were used to evaluate the adhesion: adhesion evaluation by Peel-test dry, after PBS soaking at 60 degr.C, and a Peel-test based on ASTM D1876.

From nusil.com/product/med2-4213_fast-cure-silicone-adhesive:
MED2-4213 is a two-part, translucent, thixotropic, a relatively high extrusion rate, a relatively high tear strength, a relatively fast-cure silicone adhesive. It is also substantially free of tin (Sn), reducing the requirement for atmospheric moisture to cure. It also does not comprise significant amounts of curing byproducts, such as acetic acid or methyl alcohol.
MED2-4213 is considered substantially biocompatible - the manufacturer suggests that it may be used in human implantation for a period of greater than 29 days. Typical chemical and physical properties include:

### Uncured:

| **Typical properties** | **Result** | **Standard** | **Nusil Test Method** |
|---|---|---|---|
| Appearance | Translucent | ASTM D2090 | 002 |
| Viscosity, Part A | 80000 cP (80000 mPas) | ASTM D1084, D2196 | 001 |
| Work Time | 15 hours minimum | ------ | 008 |

### Cured: 15 minutes at 150°C

| **Typical properties** | **Result** | **Standard** | **Nusil Test Method** |
|---|---|---|---|
| Specific Gravity | 1.12 | ASTM D792 | 003 |
| Durometer, Type A | 15 | ASTM D2240 | 006 |
| Tensile Strength | 1,000 psi (6.9 MPa) | ASTM D412 | 007 |
| Elongation | 800% | ASTM D412 | 007 |
| Tear Strength | 130ppi (23.0 kN/m) | ASTM D624 | 009 |

| **Property** | **Average Result** |
|---|---|
| Durometer | 15 Type A |
| Viscosity | 80,000 MPa*s (80000 cP) |
| Work Time | 15 hours |
| Tensile | 6.9 MPa (1000 psi) |
| Appearance | Translucent |
| Cure | 15 minutes / 150 °C |
| Cure System | Platinum |
| Elongation | 800 % |
| Mix Ratio | 1:1 |
| Specific Gravity | 1.12 |
| Rheology | Thixotropic / non-slump |
| Tear | 22.93 kN/m (130 ppi) |

It may be advantageous if the first (1310) and/or second (1320) encapsulation layers have/has a tensile strength in the range 6 to 8 MPa.

NuSil suggests that in many bonding applications (for a substrate comprising Aluminum, Glass, PMMA, Silicone) the use of a silicone primer to improve suitable adhesion is not required.

Use of a primer is suggested by the manufacturer when adhering to substrates comprising Polyetherimide, PEEK, Plastic, Polycarbonate, Polyimide, Polysulphone, Polyurethane, and Stainless steel.

In order to study the adhesion properties of the PDMS on LCP with different processing methods and adhesion layers, two different test substrates were used:
- TYPE 1: an LCP 3-layered substrate with ALD coating on one side
- TYPE 2: an LCP 2-layered laminated substrate with ALD coating on one side.

In general, it is advantageous to perform as few steps as possible when manufacturing an implantable electrical device - this may reduce the risk of introducing contamination or transport related issues, and it may reduce one or more costs.

A process with relatively few steps may be based around overmoulding electronics that are directly mounted on a substrate (here LCP). Depending on the hardware configuration, the PDMS used may need to adhere sufficiently well to surfaces such as:
- an ASIC passivation layer in case of wire-bonding
- a Si-substrate in case of ASIC flip-chip or ACF mounting. Si-substrate is one of the relevant interfaces when using bare-die components. Bare die integrated circuits are often made from a wafer or substrate, that is a thin slice of crystalline silicon semiconductor. To make a bare-die component, this material undergoes many microfabrication processes to become an integrated circuit, but one side will always be the raw material that was used, most often crystalline silicon. Relevant interfaces include:
- an ASIC interconnect
- Gold from wire-bonds or stud bumps
- an ACF (Anisotropic conductive film), which is frequently epoxy based, with coated gold particles, for application of a bare-die component on bond-pads.
- the substrate - in this case, a substrate comprising significant amounts of LCP.

TYPE 1 LCP substrates were prepared using one or more of the following process steps:
a) Providing a substrate: these substrates were substantially planar sheets of LCP with an average thickness of approximately 0.150 mm. The substrate comprised three layers; two 0.050mm layers of ULTRALAM^{®} 3908, separated by one 0.050mm layer of ULTRALAM 3850.

ULTRALAM^{®} 3908 LCP is available from Rogers Corporation (www.rogerscorp.com) and may be used as a bonding medium (adhesive layer) between copper, other LCP materials and/or dielectric materials. It is characterized by low and stable dielectric constant. It has a relatively low modulus, allowing relatively easy bending for flex applications, and relatively low moisture absorption.

It may be used with one or more layers of ULTRALAM^{®} 3850 LCP to create substantially adhesive-less substantially all-LCP multi-layer substrates.

Typical values for physical and chemical properties of ULTRALAM^{®} 3908 LCP include:

### Mechanical Properties

| | **Typical value** | **Units** | **Test Methods** |
|---|---|---|---|
| Dimensional Stability | MD: <0.1 | % | IPC 2.2.4 method A |
| | CMD: <0.1 | | |
| Initiation Tear Strength, min | 1.4 (3.1) | Kg (lbs) | IPC 2.4.16 |
| Tensile Strength | 216 (31) | MPa (Kpsi) | IPC 2.4.19 |
| Tensile Modulus | 2450 (355) | MPa (Kpsi) | IPC 2.4.19 |
| Thickness Variation | < +/-10 | % | ASTM-D374 |

### Thermal Properties

| | | **Test Methods** | **Units** | **Test Methods** |
|---|---|---|---|---|
| Coefficient of Thermal | | X17 | ppm/°C | IPC 2.4.41.3 |
| Expansion, CTE (30°C to 150°C) | | Y17 | | |
| | | Z:150 | | |
| Solder Float, Method B (288°C) | | PASS | | IPC 2.4.13 |
| Thermal Conductivity @ 50°C | | 0.20 | W/m/°K | ASTM D5470 |
| Melting Temperature | | 280 | °C | DSC |
| Relative Thermal Index | | | | |
| (RTI) | mechanical | 190 | °C | |
| | electrical | 240 | °C | |

### Electrical Properties

| | **Typical value** | **Units** | **Test Methods** |
|---|---|---|---|
| Dielectric Constant (10 GHz, 23°C) | 2.9 | | IPC 2.5.5.5.1 |
| Dissipation Factor (10 GHz, 23°C) | 0.0025 | | IPC 2.5.5.5.1 |
| Surface Resistivity | 1.2 X 10¹² | Mega Ohms | IPC 2.5.17 |
| Volume Resistivity | 2.6 X 10¹⁴ | Mega Ohms-cm | IPC 2.5.17 |
| Dielectric Breakdown Strength | 118 (3000) | KV/cm (V/mil) | ASTM-D-149 |

### Environmental Properties

| | **Typical value** | **Units** | **Test Methods** |
|---|---|---|---|
| Chemical Resistance | 98.7 | % | IPC 2.3.4.2 |
| Water Absorption (23°C, 24 hrs) | 0.04 | % | IPC 2.6.2 |
| Coefficient of Hydroscopic Expansion, CHE (60°C) | 4 | ppm/%RH | 60°C |
| Flammability | VTM-O | | UL-94 |

ULTRALAM^{®} 3850 is available from Rogers Corporation (www.rogerscorp.com) and is a relatively high-temperature resistant LCP. It may be provided as a double copper clad laminate for use as laminate circuit materials. The manufacturer suggests these products for use as a single layer or a multilayer substrate. ULTRALAM 3850 circuit materials are characterized by a relatively low and stable dielectric constant, and dielectric loss. It has a relatively low modulus, allowing relatively easy bending for flex applications, and relatively low moisture absorption.

It may be used with one or more layers of ULTRALAM^{®} 3908 LCP to create substantially adhesive-less substantially all-LCP multi-layer substrates.

Typical values for physical and chemical properties of ULTRALAM^{®} 3850 LCP include:

### Mechanical Properties

| | **Tvpical value** | **Units** | **Test Methods** |
|---|---|---|---|
| Dimensional Stability | MD: -0.06 | % | IPC 2.2.4 method B |
| | CMD: -0.03 | | |
| Peel Strength | 0.95 (8.52) | N/mm (lbs/in) | IPC 2.4.8 (1/2 oz. ED foil) |
| Initiation Tear Strength, min | 1.4 (3.1) | Kg (lbs) | IPC 2.4.16 |
| Tensile Strength | 200 (29) | MPa (Kpsi) | IPC 2.4.16 |
| Tensile Modulus | 2255 (327) | MPa (Kpsi) | IPC 2.4.19 |
| Density | 1.4 | gm/cm3, Typical | |

### Thermal Properties

| | | **Typical value** | **Units** | **Test Methods** |
|---|---|---|---|---|
| Coefficient of Thermal | | X:17 | ppm/°C | IPC 2.4.41.3 |
| Expansion, CTE | | Y:17 | | |
| (30°C to 150°C) | | Z:150 | | |
| Solder Float, Method B | | PASS | | IPC 2.4.13 |
| (288°C) | | | | |
| Melting Temperature | | 315 | °C (Typical) | DSC |
| Relative Thermal Index | | | | |
| (RTI) | mechanical | 190 | °C | |
| | electrical | 240 | °C | |
| Thermal Conductivity @ 50°C | | 0.2 | W/m/°K | ASTM C518 |
| Thermal Coefficient of Σr, -50°C to 150°C | | (+)24 | ppm/°C | IPC 2.5.5.5, 8 GHz |

### Electrical Properties

| | **Typical value** | **Units** | **Test Methods** |
|---|---|---|---|
| Dielectric Constant, 10 GHz, 23°C (Process) | 2.9 | | IPC 2.5.5.5.1 |
| Dielectric Constant, 10 GHz, 23°C (Design) | 3.14 | | Differential Phase Length Method |
| Dissipation Factor (10 GHz, 23°C) | 0.0025 | | IPC 2.5.5.5.1 |
| Surface Resistivity | 1×10¹⁰ | Mega Ohms | IPC 2.5.17 |
| Volume Resistivity | 1×10¹² | Mega Ohms-cm | IPC 2.5.17 |
| Dielectric Breakdown Strength | 1378 (3500) | KV/cm (V/mil) | ASTM-D-149 |

### Environmental Properties

| | **Typical value** | **Units** | **Test Methods** |
|---|---|---|---|
| Chemical Resistance | 98.7 | % | IPC 2.3.4.2 |
| Water Absorption (23°C, 24 hrs) | 0.04 | % | IPC 2.6.2 |
| Coefficient of Hydroscopic Expansion, CHE (60°C) | 4 | ppm/%RH | 60°C |
| Flammability | VTM-0 | | UL-94 |

TYPE 1 LCP substrates were further prepared using one or more of the following process steps:
b1) an optional pre-cleaning of at least a portion of the substrate using IPA, followed by drying. Another suitable alcohol may also be used.
b2) Applying an adhesion coating: using ALD, a coating was applied to an outer surface of the substrate - in this case a surface comprising ULTRALAM^{®} 3908 LCP. Ten alternating layers of approximately 5nm Al2O3 and of approximately 5nm HfO2, resulted in an approximately 100nm multilayer. The extent of the ALD coating was approximately the same as the extent of the substrate. The ALD coating was applied using the PICOSUN^{®} R-200 Advanced ALD reactor described above. It was applied at a temperature substantially lower than the melting temperature of the LCP. For these TYPE 1 LCP substrates, it was applied at approximately 125 degr C after an optional stabilization time of approximately 90 minutes.

For comparison, this step was omitted for some of the samples (in other words, the PDMS was applied directly to the LCP).

c) Cleaning at least a portion of the adhesion coating: as preparation for the PDMS coating, an optional ten-minute ozone (O3) plasma treatment was performed to clean the ALD surface. The PDMS was applied within fifteen minutes from the ozone cleaning. For comparison, some samples were not cleaned before the PDMS coating was applied

UV O3 (ozone) plasma cleaning is suitable for dry, non-destructive atomic cleaning and removal of organic contaminants. It uses intense 185 nm and 254 nm ultraviolet light. In the presence of oxygen, the 185 line produces Ozone and while the 254 line excites organic molecules on the surface. This combination drives the rapid destruction and decimation of organic contaminants.

d) Applying an encapsulation coating: a PDMS coating of approximately 500 um to 1000 um of MED2-4213 was applied on top of the ALD coating. A syringe was filled with MED2-4213, and mixed & degassed at relatively high speed (2500 rpm) for three minutes. It was cured at 150 degr C for 10min and post-cured at 80 degr C for 24 hours. The extent of the PDMS coating was less than the extent of the ALD coating, whereby the ALD coating was exposed (not covered by encapsulant) close to the edge of the substrate. After applying the PDMS on the substrate, the substrate was placed on the PTFE (polytetrafluorethylene)-coated pre-heated plate, and a weight was pressed on top of it.

So, six samples of TYPE 1 LCP were prepared:

| | **Nr of samples** | **ALD coating** | **Cleaning** | **PDMS coating** |
|---|---|---|---|---|
| 1.1 | Two | None | None | MED2-4213 |
| 1.2 | Two | Ten x (5 nm Al2O3 + 5nm HfO2) | None | MED2-4213 |
| 1.3 | Two | Ten x (5 nm Al2O3 + 5nm HfO2) | O3-plasma (10 min) | MED2-4213 |

A Pass/ Fail test was defined for the TYPE 1 LCP substrates by hand:
- after curing the PDMS, a dry Peel-test was performed (no soaking) and the degree of delamination was noted
- after the dry Peel-test, the samples were soaked in a PBS solution at 60 degr C for 1 day, 1 week, and 4 weeks, and the Peel-test was repeated to determine a second degree of delamination.

Three degrees of delamination were defined:
- Delamination: the PDMS can be removed relatively easily from the substrate
- Partial delamination: the PDMS can be removed relatively easily in some areas, but sticks relatively well in other areas
- Adhesion: the PDMS does not substantially delaminate

Phosphate-buffered saline (abbreviated PBS) is a buffer solution commonly used in biological research. It is a water-based salt solution containing disodium hydrogen phosphate, sodium chloride and, in some formulations, potassium chloride and potassium dihydrogen phosphate. The buffer helps to maintain a constant pH. The osmolarity and ion concentrations of the solutions are selected to match those of the human body (isotonic).

| **Samples** | **Dry** | **Soak after 24h @60°C PBS** | **Soak after 1 week @60°C PBS** | **Soak after 4 weeks @60°C PBS** |
|---|---|---|---|---|
| 1.1 | Delamination | Not applicable | Not applicable | Not applicable |
| 1.2 | Adhesion | Partial Delamination | Partial Delamination | Partial Delamination |
| 1.3 | Adhesion | Adhesion | Adhesion | Adhesion |

### Samples 1.1: in general, PDMS has a low degree of adhesion to LCP

Samples 1.2: the PDMS could not be peeled from the surface in dry state. After twenty-four hours of soaking, part of the PDMS could be peeled from the substrate, although no moisture filled voids were observed. After peeling away some of the PDMS, the rest stuck so well to the substrate it could not be peeled off any further, not even after 1 or 2 weeks of additional soaking. It was suspected that the initial delamination was due to local contamination during PDMS processing or processing issues.

Samples 1.3: these samples showed good adhesion. No delamination was achieved in dry and wet conditions until after two weeks of testing.

### Conclusions:

- In general, PDMS had a low degree of adhesion to LCP without any adhesion layer (samples 1.1).
- In samples 1.2 (coated with ALD and then encapsulated with PDMS without O3 cleaning), the PDMS could not be peeled from the surface in dry state. After 24 hours of soaking, part of the PDMS could be peeled from the substrate, although no moisture filled voids were observed. After peeling away some of the PDMS, the rest stuck so well to the substrate it could not be peeled off any further, not even after two weeks of additional soaking. Samples with a more conformal ALD multilayer showed good adhesion even after two weeks of soaking.

- Samples 1.3 (coated with ALD and encapsulated with PDMS after an O3 cleaning step) showed the highest degree of adhesion. No substantial delamination was achieved in dry or wet conditions (after 2 weeks of soaking)
- An Al2O3/HfO2 multilayer ALD layer with a total average thickness of approximately 50nm to 200nm, preferably approximately 100nm, may provide an advantageous intermediate adhesion layer between LCP and the PDMS polymer.,

TYPE 2 LCP laminated substrates were prepared using one or more of the following process steps:
a) Providing a substrate: these substrates were laminated sheets of LCP, with an average thickness of approximately 0.110 mm. The substrate comprised four layers; one outer layer of copper connection pads, one 0.050 mm layer of ULTRALAM^{®} 3850, one inner layer of one or more copper conductors, and one 0.025mm layer of ULTRALAM 3908:
   a1) an approximately 50um-thick sheet of LCP ULTRALAM^{®} 3850, clad on a first surface with a first copper layer. This first copper layer was approximately 18um-thick. The first copper layer was configured and arranged to form copper connection pads, for example by masking and etching, which may be considered to be comprised in an outer surface of the laminated substrate;
   a2) the ULTRALAM^{®} 3850 was further clad with a further copper layer. This second layer was approximately 18um-thick. Optionally, it may be configured and arranged to form one or more conductors, for example by masking and etching, which may be considered to be comprised in an inner surface of the laminated substrate. If no inner conductors are required, the further copper layer may be omitted or completely removed. ;
   a3) an approximately 25um-thick sheet of LCP ULTRALAM^{®} 3908, bonded to the inner surface of the ULTRALAM^{®} 3850 layer, and further bonded to the one or more conductors.

Optionally, the laminated sheets may be substantially planar, which is according to the claimed invention.
b) Applying an adhesion coating:
   b1) an optional pre-cleaning of at least a portion of the substrate using IPA, followed by drying. Another suitable alcohol may also be used.
   b2) using ALD, a coating was applied to an outer surface of the substrate - in this case a surface comprising ULTRALAM^{®} 3908 LCP. It was not the outer surface of the substrate comprising one or more connection pads. Ten alternating layers of approximately 5nm Al2O3 and of approximately 5nm HfO2 resulted in an approximately 100nm multilayer. The ALD coating was applied using the PICOSUN^{®} R-200 Advanced ALD reactor described above. It was applied at a temperature substantially lower than the melting temperature of the LCP. For these TYPE 1 LCP substrates, it was applied at approximately 125 degr C after an optional stabilization time of approximately 90 minutes. The extent of the ALD coating was approximately the same as the extent of the substrate.
   b3) Applying an adhesion improver: MED-166 from NuSil is a specially formulated primer which is suggested by the manufacturer to improve adhesion of PDMS to various substrates including: rigid plastics, and other silicone materials. The manufacturer suggests that it is suitable for use in human implantation for a period of greater than 29 days.
c) Cleaning at least a portion of the adhesion coating before encapsulation:
   c1) Option 1: cleaning using ethanol, followed by drying for 4 hours at 70°C. Another suitable alcohol may also be used.
   c2) Option 2: exposing the ALD surface to a plasma comprising O2.

O2 (oxygen) plasma refers to any plasma treatment performed while actively introducing oxygen gas to the plasma chamber. Oxygen plasma is created by utilizing an oxygen source on a plasma system.

Additionally or alternatively, ozone (O3) may be used.

d) Applying an encapsulation coating:
d1) Applying an encapsulation mask to simplify testing: a strip of Kapton tape (10mm wide) was applied to one edge to mask a small section to which the pull-tester is to be clamped during the Peel-test.
d2) Applying an encapsulation coating: a PDMS coating of approximately 500 um to 1000 um of MED2-4213 was applied on top of the ALD coating. Vacuum centrifugal casting at 100 degr. C used with PTFE-coated molds under a relatively low vacuum, for example 800 - 900 Pa (8 to 9 mbar) -vacuum centrifugal casting was used to reduce the risk of air inclusion in the PDMS. In general, applying a vacuum may be advantageous in improving the application to an adhesion coating of the encapsulation of a PDMS having an average viscosity in the range 55000 to 100000 cP (mPas) for a significant time period.

The extent of the PDMS coating was approximately the same as the extent of the ALD coating. After removing the Kapton tape, a strip of approximately 10mm wide was provided where the PDMS was not attached to the ALD coating.

e) Performing further processing: the coated substrate of approximately 100x75mm area was cut into 7 pieces of approximately 100×10mm for Peel-testing. Each piece had an area of approximately 10×10mm without the PDMS coating at is edge due to Kapton tape removal.

So, fifteen samples of type (2) were prepared:

| | **Nr of samples** | **ALD coating** | **Primer** | **Cleaning** | **PDMS coating** |
|---|---|---|---|---|---|
| 2.1 | Three | None | | Ethanol | MED2-4213 |
| 2.2 | Three | None | MED-166 | Ethanol | MED2-4213 |
| 2.3 | Three | 10x (5 nm Al2O3 + 5nm HfO2) | None | Ethanol | MED2-4213 |
| 2.4 | Three | None | None | O2 plasma treatment | MED2-4213 |
| 2.5 | Three | 10x (5 nm Al2O3 + 5nm HfO2) | None | O2 plasma treatment | MED2-4213 |

Peel-test according to ASTM D1876 was adapted for testing the TYPE 2 LCP or laminated substrates). A Peel-tester was used to measure the lamination force.

### 4. Peel-test results

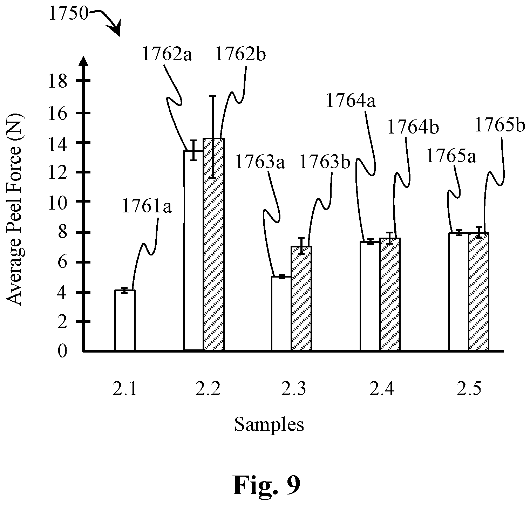

FIG. 9 depicts a graph 1750 comparing the average pull force under dry (not soaked) conditions with the average pull forces after 24 hours of soaking at 60 degr. C in PBS. The samples were coated with PDMS using different processes.

Average peel force is plotted along the vertical (Y) axis from 0 to 18 N, and the results are indicated for the different samples along the horizontal (X) axis. To simplify interpretation, the order of the samples chosen is numerical: from left to right, samples 2.2, 2.2, 2.3, 2.4 and 2.5.

For each sample, the vertical length of each bar indicates the average peel force in Newtons (N). For each bar, an "I" shaped line is also depicted to indicate the variation measured in the pull force values used to determine the average. For each sample, an unfilled bar is depicted on the left-hand side showing the average pull force under dry conditions, and a hatched bar on the right-hand side showing the average pull force after 24 hours of soaking at 60 degr. C in PBS.

For sample 2.1, an unfilled bar 1761a is depicted of approx. 4N, with a relatively small degree of variation. No value after soaking is depicted.

For sample 2.2, an unfilled bar 1762a is depicted of approx. 13N, with an average degree of variation. A hatched bar 1762b is depicted of approx. 14N, with a relatively high degree of variation.

For sample 2.3, an unfilled bar 1763a is depicted of approx. 5N, with a relatively small degree of variation. A hatched bar 1763b is depicted of approx. 7N, with an average degree of variation.

For sample 2.4, an unfilled bar 1764a is depicted of approx. 7N, with a relatively small degree of variation. A hatched bar 1764b is depicted of approx. 7.5N, with an average degree of variation.

For sample 2.5, an unfilled bar 1765a is depicted of approx. 8N, with a relatively small degree of variation. A hatched bar 1765b is depicted of approx. 8N, with an average degree of variation.

The average peel forces measured were:

| **Samples** | **Average Peel Force (N) - Drv** | **Average Peel Force (N) - After 24 hours soaking in PBS at 60 degr. C** |
|---|---|---|
| 2.1 | 4.05 (761a) | Not applicable |
| 2.2 | 13.38 (762a) | 14.22 (762b) |
| 2.3 | 5.23 (763a) | 7.29 (763b) |
| 2.4 | 7.31 (764a) | 7.56 (764b) |
| 2.5 | 8.31 (765a) | 8.32 (765b) |

It appears that a stable over molding encapsulation process was achieved, showing substantially none, or very few, air bubbles in the PDMS. Substantial delamination of the LCP/PDMS interface was observed on 3 out of 7 samples directly after over molding. For this reason, the Peel-test was applied to get a more qualitative measure of the adhesion strength.

Samples 2.1: without additional priming or cleaning, the PDMS had a very low degree (approx. 4N - 1761a) of adhesion to LCP.

Samples 2.2: substrates with a primer appeared to have a relatively high degree of adhesion (approx. 13N - 1762a - compared to approx. 4N - 1761a). During the test, some regions had a higher degree of adhesion, which resulted in the PDMS rupturing before peeling the samples completely. The average pull force after the soaking test appeared to be higher at approx. 14N - 1762b, but a relatively high degree of deviation was also observed.

Samples 2.3: by adding an ALD multilayer, specifically the HfO2-Al2O3 multilayer ending with HfO2, the degree of dry adhesion appeared improved (from approx. 4N - 1761a - to approx. 5N - 1763a). The results under dry conditions - 1763a - appears to have a very low degree of deviation. The average pull force after the soaking test appeared to be higher at approx. 7N - 1763b.

Samples 2.4: O2-plasma activation also appeared to increase the adhesion (approx. 7N - 1764a - compared to approx. 4N - 1761a).. The average pull force after the soaking test appeared to be slightly higher at approx. 7.5N - 1764b.

Samples 2.5: plasma activation appeared to further improve the degree of adhesion (approx. 8N - 1765a - compared to approx. 4N - 1761a). The average pull force after the soaking test appeared to be approximately the same at 8N - 1765b. A small increase in deviation - 1765b - was observed after soaking.

### Conclusions:

- A relatively high degree of adhesion was observed using a primer - for example, MED-166 from NuSil may be used. But it may be less-preferred in some uses. In particular, for implantable devices, it is advantageous to use materials that are significantly biocompatible and more preferably materials that are substantially biocompatible (have a high degree of biocompatibility). Although the manufacturer of MED-166 suggests that it is suitable for implantation for more than 29 days, primers are often epoxy adhesives which use volatile solvents. This may increase the risk of contamination due to insufficient evaporation and/or require additional process steps to ensure sufficient solvent removal.

In addition, for implantable devices, a high degree of quality control is often required to limit the risk of defects. Primers must typically be applied using a spray coating process, which may be difficult to perform with a high degree of reliability. It is believed that such reliability issues were the cause of the partial delamination observed.
- An Al2O3/HfO2 multilayer ALD layer with a total average thickness of approximately 50nm to 200nm, preferably approximately 100nm, may provide an advantageous intermediate adhesion layer between LCP and the PDMS polymer. In general, a ceramic layer with a thickness of less than ,
- the ALD coating used improved adhesion of PDMS to LCP using materials suitable for use in an implantable electrical device.
- the ALD multilayer stack not only improved the adhesion, but also improved the barrier property to protect a surface region against moisture ingress. This is preferred over a primer because the primer typically has a high degree of permeability for moisture.

Based upon the improved adhesion between a PDMS and surfaces comprising a significant amount of Pt, SiO2 and an LCP, an adhesion layer comprising a ceramic material may be advantageously used for a wide range of substrate materials. In particular, adhesion of a PDMS may be improved where a first surface 1410 and/or second surface 1420 comprises a significant amount of a substance selected from the group comprising: a Liquid-Crystal Polymer (LCP), a polyimide, Parylene-C, SU-8, a polyurethane, or any combination thereof. These substances may be comprised in a flexible substrate.

Where appropriate, a substrate comprising other materials may thus be provided with a layer of such a material to improve adhesion to the HfO2 ALD layer.

The skilled person will also realize that adhesion may be improved by optionally or additionally applying a conformal coating to such a substrate, for example with an ALD process, applying a layer of SiO2 (silicon dioxide).

PDMS is, in general, a silicone rubber, with siloxane as the basic repeating unit. Methyl groups are substituted by a variety of other groups, for example, phenyl, vinyl or trifluoropropyl groups, depending on the type of PDMS, enabling the linkage of organic groups to an inorganic backbone.

Based upon the improved adhesion to a PDMS using one or more adhesion layers comprising HfO2 and/or Al2O3, an adhesion layer comprising a suitable ceramic material may be advantageously used for a wide range of substrate materials.

Suitable ceramic surfaces are relatively rich in hydroxyl groups. It is believed that the high degree of adhesions is due to oxygen in suitable PDMS-types can form strong bonds with the hydroxyl groups on the suitable ceramic surface. This may be chemical bonding, hydrogen-bridge bonding or some combination.

Suitable ceramics include:
- carbides, such as silicon carbide (SiC);
- oxides, such as aluminum oxide (Al2O3);
- nitrides, such as with silicon (SixNy or SiNxOy) and, in particular, silicon nitride (Si3N4); and
many other materials, including the mixed oxide ceramics that can act as superconductors.

In particular, adhesion of a PDMS may be improved where the ceramic material is selected from the group comprising: HfO2, Al2O3, Ta2O3, TiO2, and any combination thereof.

It is also expected that Diamond-like carbon may be advantageously used to improve adhesion.

Adhesion may be further improved by activating the surface of the ceramic layer - for example, by applying an alcohol, in particular ethanol; using a plasma comprising O3 (Ozone) and/or comprising O2; treating with a silane; or any combination thereof.

An adhesion layer may be a bi-layer or multilayer, in which one or more layer may be configured and arranged for a relatively high degree of adhesion, and one or more layers may be configured and arranged for a relatively high degree of corrosion resistance (impermeability).

For example, it is believed that a layer comprising Al2O3 provides a relatively high degree of adhesion. For example, it is believed that a layer comprising HfO2 provides a relatively high degree of corrosion resistance.

FIG. 5 and FIG. 6 also depict examples of nerves that may be stimulated using one or more suitably configured improved medical devices 1110, 1111, configured to provide neurostimulation to treat, for example, headaches, chronic headaches or primary headaches. In particular, if the substrate is substantially flexible (or conformable), it may conform better to the curved surfaces of the head and/or skull. This means that the comfort to the user of an implantable medical device 1110, 1111 may be increased by applying one or more of the features described above for improving conformance.

In many cases, these will be the approximate locations 810, 820, 830, 840 for the one or more implantable medical devices 110, 111.

For each implant location, 810, 820, 830a/b, 840a/b a separate stimulation device 110, 111 may be used. Where implant locations 810, 820, 830a/b, 840a/b are close together, or even overlapping a single stimulation device 110, 111 may be configured to stimulate at more than one implant location 810, 820, 830a/b, 840a/b.

A plurality of implantable medical devices 110, 111 may be operated separately, simultaneously, sequentially or any combination thereof to provide the required treatment.

FIG 7 depict further examples of nerves that may be stimulated using one or more suitably configured improved implantable medical devices 110, 111 to provide neurostimulation to treat other conditions.

The descriptions thereof herein should not be understood to prescribe a fixed order of performing the method steps described therein. Rather the method steps may be performed in any order that is practicable. Similarly, the examples used to explain the algorithm are presented as non-limiting examples, and are not intended to represent the only implementations of these algorithms. The person skilled in the art will be able to conceive many different ways to achieve the same functionality as provided by the embodiments described herein.

For example, one or more features that improve conformance may be applied to embodiments that are configured and arranged for improved encapsulation. In some embodiments, it may be advantageous to apply features that improve encapsulation but reduce conformance.

For example, one or more features that improve encapsulation may be applied to embodiments that are configured and arranged for improved conformance. In some embodiments, it may be advantageous to apply features that improve conformance but reduce encapsulation.

Many types of implantable distal ends of stimulation devices are depicted. But this does not exclude that the rest of the device is implanted. This should be interpreted as meaning that at least the electrode section of the distal end is preferably configured and arranged to be implanted.

In a non-limiting example,
- one or more electrodes of the first type 200a, 200b, 1220 are comprised in the first surface 310, 1410 and one or more electrodes of the second type 400a, 400b, 1220 are comprised in the second surface 320, 1420; or
- one or more electrodes of the first type 200a, 200b, 1220 are comprised in the first surface 310, 1410 and one or more electrodes of the second type 400a, 400b, 1220 are also comprised in the first surface 310, 1410; or
- one or more electrodes of the first type 200a, 200b, 1220 are comprised in the second surface 320, 1420 and one or more electrodes of the second type 400a, 400b, 1220 are comprised in the first surface 310, 1410; or
- one or more electrodes of the first type 200a, 200b, 1220 are comprised in the second surface 320, 1420 and one or more electrodes of the second type 400a, 400b, 1220 are also comprised in the second surface 320, 1420; or
- any combination thereof.

By providing relatively larger higher electrode 200, 400, 1220 surfaces, stimulators 100, 101, 102, 103, 104, 105, 1100, 1101, 1102 may be operated at a lower energy / lower power. This may be advantageous in applications where high frequency and/or burst stimulation is used.

High frequency operation may require more energy to be provided by the pulse generator 500. In applications where energy / power is critical, such as, in a non-limiting example, if an increased operating lifetime is desired from a power source for the pulse generator 500), any reduction in required power may be advantageous. High frequency operation may be considered as generating electrical stimulation pulses with a frequency of 1000 Hz or more, preferably 1500 Hz or more, more preferably 2000 Hz or more, yet more preferably 2500 Hz or more.

In an embodiment, experiments with burst stimulation have been performed such as Burst Occipital Nerve Stimulation for Chronic Migraine and Chronic Cluster Headache by Garcia-Ortega et al, Neuromodulation 2019; 22: 638-644, DOI: 10.1111/ner. 12977.

For burst operation, the pulse generator 500 is further configured and arranged to generate electrical stimulation pulses in groups of stimulation pulses.

In a non-limiting example, groups (or bursts) of stimulation pulses may comprise 2 to 10 pulses, more preferably 2 to 5 stimulation pulses. Stimulation pulses in a group may have a repetition frequency of more than 500 Hz, typically 1000Hz or more. Groups may be repeated at more than 5 Hz, typically 40 Hz or more.

As with high frequency operations, burst operation may require more energy to be provided by the pulse generator 500, and any reduction in required power may be advantageous.

Additionally, the speed of charge-balance recovery may also increase with a lower impedance. By using a relatively thin-foil substrate 300, 1400, stimulation between an electrode of the first type 200, 1220 comprised in one surface 310, 1410, 320, 1420 and an electrode of the second type 400, 1220 comprised in the other surface 310, 1410, 320, 1420, the current path in tissue is relatively short, reducing impedance.

Similarly, using a substrate 300, 1400 and stimulation between an electrode of the first type 200, 1220 comprised in one surface 310, 1410, 320, 1420 and an adjacent electrode of the second type 400, 1220 comprised in the same surface 310, 1410, 320, 1420 provide a relatively short path through tissue.

### REFERENCE NUMERALS:

- 100, 101, 102: implantable stimulators
- 103, 104, 105: further embodiments of implantable stimulators
- 200a, 200b: one or more stimulation electrodes
- 250: one or more stimulation electrical interconnection layers
- 300: a conformable foil-like substrate
- 400a, 400b: one or more return electrodes
- 500: a pulse generator
- 600: a longitudinal axis
- 700: a first transverse axis
- 750: a second transverse axis
- 810: location for left supraorbital nerve or cortical stimulation
- 820: location for right supraorbital stimulation
- 830a/b: location for left occipital nerve stimulation
- 840a/b: location for right occipital nerve stimulation
- 850: location for deep brain stimulation
- 860: location for vagus nerve, carotid artery, carotid sinus, phrenic nerve or hypoglossal stimulation
- 865: location for cerebral spinal cord stimulation
- 870: location for peripheral nerve stimulation
- 875: location for spinal cord stimulation
- 880: location for gastric stimulation
- 885: location for sacral & pudendal nerve stimulation
- 890: location for sacral neuromodulation
- 895: location for fibular nerve stimulation
- 910: left supraorbital nerve
- 920: right supraorbital nerve
- 930: left greater occipital nerve
- 940: right greater occipital nerve
- 1100, 1101, 1102: improved implantable electrical or electronic devices
- 1110, 1111: improved implantable medical devices
- 1130: a test substrate
- 1210: one or more electrical conductor
- 1220: one or more stimulation electrodes
- 1230: one or more sensors
- 1300: a further biocompatible encapsulation layer
- 1310: a first biocompatible encapsulation layer
- 1320: a second biocompatible encapsulation layer
- 1330: a third biocompatible encapsulation layer
- 1400: a substrate
- 1430: a silicon substrate
- 1435: a SiO2 (silicon dioxide) layer
- 1410: a first surface
- 1420: a second surface
- 1500: a further adhesion layer
- 1510: a first adhesion layer
- 1520: a second adhesion layer
- 1530: a third adhesion layer
- 1700: Bode plots presented as impedance magnitude
- 1701: Impedance magnitude for a bare IDC with exposed Pt metal
- 1702: Impedance magnitude for an IDC coated with HfO2 ALD
- 1703: Impedance magnitude for an IDC coated with an ALD-PDMS bilayer
- 1710: Bode plots presented as phase angle
- 1711: Phase angle for a bare IDC with exposed Pt metal
- 1712: Phase angle for an IDC coated with HfO2 ALD
- 1713: Phase angle for an IDC coated with an ALD-PDMS bilayer
- 1720: Adhesion monthly performance presented as impedance magnitude
- 1722a, 1722b: Impedance magnitude for IDC's coated with HfO2 ALD
- 1723a, 1723b: Impedance magnitude for IDC's coated with an ALD-PDMS bilayer
- 1730: Adhesion monthly performance presented as phase angle
- 1732a, 1732b: Phase angle for IDC's coated with HfO2 ALD
- 1733a, 1733b: Phase angle for IDC's coated with an ALD-PDMS bilayer810 location for left supraorbital nerve or cortical stimulation
- 1750: Graph comparing the average pull force under dry conditions and after soaking
- 1761: Average peel force for sample 2.1
- 1762: Average peel force for sample 2.2
- 1763: Average peel force for sample 2.3
- 1764: Average peel force for sample 2.4
- 1765: Average peel force for sample 2.5

## Claims

1. An implantable stimulator (100, 101, 102, 103, 104, 105, 1110, 1111) comprising:
- a pulse generator (500) configured to generate at least one stimulation pulse;
- a conformable foil-like substrate (300, 1400) having a longitudinal axis (600) extending from the pulse generator (500) to a distal end of the substrate (300, 1400), the substrate (300, 1400) comprising one or more adjacent polymeric substrate layers, the substrate having a first (310, 1410) and second (320, 1420) substantially planar surface; and
- an electrode array (200, 400, 1220), proximate the distal end (300, 1400), having a first (200, 1220) and second (400, 1220) electrode comprised in the first (310, 1410) or second surface (320, 1420), located along the conformable portion of the substrate (300, 1420), each electrode (200, 400, 1220) in operation being configurable for transferring treatment energy, in use, to and/or from human or animal tissue;
the implantable stimulator (100, 101, 102, 103, 104, 105, 1110, 1111) further comprising:
- one or more electrical interconnections (250, 1210), between the pulse generator (500) and the first (200, 1220) and the second (400, 1220) electrodes, for transferring electrical energy as one or more electrical treatment stimulation pulses to the coupled first electrodes (200) and/or the second electrodes (400);
wherein the one or more electrical interconnections (250, 1210) are positioned between the first (310, 1410) and second (320, 1420) surfaces of the substrate (300, 1400);
wherein the conformable foil-like substrate (300, 1400) has a maximum thickness of 0.5 millimeter or less, proximate the first and second electrodes (200, 400, 1220), , the thickness being determined by a perpendicular distance between corresponding points on the first (310, 1410) and second outer planar surfaces (320, 1420), and
wherein the substrate (300, 1400) and the pulse generator (500) are embedded in one or more flexible bio-compatible encapsulation layers comprising Polydimethylsiloxane (PDMS).

2. The implantable stimulator according to any preceding claim, the implantable stimulator (100, 101, 102, 103, 104, 105, 1110, 1111) further comprising an adhesion layer (1500, 1510, 1520) adjacent to at least part of the substrate (300, 1400), the adhesion layer (1500, 1510, 1520) comprising a ceramic material.

3. The implantable stimulator according to claim 2, wherein the substrate (300, 1400) comprises more than one adjacent substrate layer and the adhesion layer (1500, 1510, 1520) is between substrate layers.

4. The implantable stimulator according to any one of claims 2 to 3, the implantable stimulator (100, 101, 102, 103, 104, 105, 1110, 1111) further comprising one or more additional adhesion layers (1500, 1510, 1520), the one or more adhesion layers (1500, 1510, 1520) comprising a ceramic material.

5. The implantable stimulator according to claim 4, wherein the one or more additional adhesion layers (1500, 1510, 1520) are between substrate layers.

6. The implantable stimulator according to any preceding claim, wherein the conformable foil-like substrate (300, 1400) has a maximum thickness, proximate the electrode array (200, 400, 1220), of 0.3 millimeters, or of 0.2 millimeters, or of 0.1 millimeters.

7. The implantable stimulator according to any preceding claim, wherein the stimulator (100, 101, 102, 103, 104, 105, 1110, 1111) has a maximum thickness, proximate the pulse generator (500), of 5 millimeters, or of 4 millimeters, or of 3 millimeters.

8. The implantable stimulator according to any preceding claim, where:
the substrate (300, 1400) has a surface comprised in a plane with a transverse extent substantially perpendicular to the longitudinal axis (600);
wherein the conformable portion of the substrate (300, 1400) has a maximum planar width, the width being determined by a perpendicular distance between corresponding points on outer surfaces edges of the substrate (300, 1400) along the transverse extent; and
wherein the ratio of maximum planar width to a maximum thickness proximate the at least two electrodes (200, 400) is equal to or more than 7: 1, or is equal to or more than 10:1, or is equal to or more than 15:1, is equal to or more than 30:1, or is equal to or more than 50:1.

9. The implantable stimulator according to any preceding claim, wherein the conformable portion of the substrate (300, 1400) comprises one or more layers of the Liquid Crystal Polymer (LCP).

10. The implantable stimulator according to any preceding claim, wherein:
- the pulse generator (500) is located along a first portion of the substrate (300, 1400);
- the electrode array (200, 400, 1220) is located along a second, conformable Liquid Crystal Polymer (LCP) portion of the substrate (300, 1400);
wherein the plurality of electrical interconnections (250, 1210) are positioned on a first conformable LCP layer of the substrate (300, 1400) using electro-plating and/or a semiconductor deposition technique and at least one second conformable LCP layer of the substrate (300, 1400) is secured to the first layer so as to cover the plurality of electrical interconnections (250, 1210);
the implantable stimulator (100, 101, 102, 103, 104, 105, 1110, 1111) further comprising:
- one or more adhesion layers (1500, 1510, 1520) adjacent to the substrate (300, 1400); and
- an encapsulation layer (1300, 1310, 1320) covering the first portion of the substrate (300, 1400), the encapsulation layer comprising Polydimethylsiloxane (PDMS);
wherein the thickness of the substrate (300, 1400) along the second portion is equal to or less than 0.2 millimeters;
wherein a thickness of the stimulator along the first portion is equal to or less than 3 millimeters;
wherein the pulse generator (500) comprises an energy receiver configured to wirelessly receive energy from an energy transmitter.

11. The implantable stimulator according to any preceding claim, wherein the stimulator (100, 101, 102, 103, 104, 105, 1110, 1111) further comprises:
- an encapsulation layer (1300, 1310, 1320) at least partially covering the substrate (300, 1400); and
- a bio-compatible adhesion layer (1500, 1510, 1520) between the encapsulation layer (1300, 1310, 1320) and the substrate (300, 1400) in at least one location.

12. The implantable stimulator according to claim 11, wherein the encapsulation layer (1300, 1310, 1320) covers at least part of the conformable portion of the substrate (300, 1400), and wherein the adhesion layer (1500, 1510, 1520) is between the encapsulation layer (1300, 1310, 1320) and the at least part of the conformable portion of the substrate (300, 1400).

13. The implantable stimulator according to any one of claims 11 to 12, , wherein the adhesion layer (1500, 1510, 1520) comprises a ceramic material, the ceramic material being selected from the group consisting of HfO2, Al2O3, Ta2O3, Si3N4, TiO2, or any combination thereof.

14. The implantable stimulator according to any one of claims 11 to 13, wherein the conformable part of the substrate (300, 1400) has a Young's modulus in the range 2500 to 3600 MPa.

15. The implantable stimulator according to any one of claims 11 to 14, , wherein the encapsulation layer (1300, 1310, 1320) has a tensile strength in the range 6 to 8 MPa.

16. The implantable stimulator according to any one of claims 11 to 15, wherein a ceramic portion of the adhesion layer (1500, 1510, 1520) has an average thickness in the range of 25nm to 200nm.

17. The stimulator according to any one of claims 1 to 16, configurable to stimulate one or more nerves, one or more muscles, one or more organs, spinal cord tissue, brain tissue, one or more cortical surface regions, one or more sulci, and any combination thereof.

18. The stimulator according to any one of claims 1 to 16, configurable to treat headaches, chronic headaches, primary headaches, incontinence, occipital neuralgia, sleep apnea, hypertension, gastro-esophageal reflux disease, an inflammatory disease, limb pain, leg pain, back pain, lower back pain, phantom pain, chronic pain, epilepsy, an overactive bladder, poststroke pain, obesity, an autoimmune disorder, rheumatoid arthritis, inflammatory bowel disease, Crohn's disease, and any combination thereof.

## Patentansprüche

1. Implantierbarer Stimulator (100, 101, 102, 103, 104, 105, 1110, 1111), der Folgendes umfasst:
- einen Impulsgenerator (500), der zur Erzeugung von mindestens einem Stimulationsimpuls konfiguriert ist;
- ein anpassbares folienartiges Substrat (300, 1400), das eine Längsachse (600) aufweist, die sich von dem Impulsgenerator (500) zu einem distalen Ende des Substrats (300, 1400) erstreckt, wobei das Substrat (300, 1400) eine oder mehrere angrenzende polymere Substratschichten umfasst, wobei das Substrat eine erste (310, 1410) und zweite (320, 1420), im Wesentlichen planare Oberfläche aufweist; und
- eine Elektrodenanordnung (200, 400, 1220) nahe dem distalen Ende (300, 1400), die eine erste (200, 1220) und zweite (400, 1220) Elektrode aufweist, die in der ersten (310, 1410) oder zweiten Oberfläche (320, 1420) enthalten sind, sich entlang des anpassbaren Teils des Substrats (300, 1420) befinden, wobei jede Elektrode (200, 400, 1220) in Betrieb zur Übertragung von Behandlungsenergie beim Einsatz zu und/oder von menschlichem oder tierischem Gewebe konfigurierbar ist;
wobei der implantierbare Stimulator (100, 101, 102, 103, 104, 105, 1110, 1111) weiter Folgendes umfasst:
- eine oder mehrere elektrische Verbindungen (250, 1210) zwischen dem Impulsgenerator (500) und den ersten (200, 1220) und den zweiten (400, 1220) Elektroden zur Übertragung von elektrischer Energie als ein oder mehrere elektrische Behandlungsstimulationsimpulse zu den verbundenen ersten Elektroden (200) und/oder den zweiten Elektroden (400);
wobei die eine oder mehreren elektrischen Verbindungen (250, 1210) zwischen der ersten (310, 1410) und zweiten (320, 1420) Oberfläche des Substrats (300, 1400) positioniert sind;
wobei das anpassbare folienartige Substrat (300, 1400) eine maximale Dicke von 0,5 Millimeter oder weniger, nahe den ersten und zweiten Elektroden (200, 400, 1220), aufweist, wobei die Dicke durch einen senkrechten Abstand zwischen entsprechenden Punkten auf der ersten (310, 1410) und zweiten äußeren planaren Oberfläche (320, 1420) bestimmt wird, und
wobei das Substrat (300, 1400) und der Impulsgenerator (500) in einer oder mehreren flexiblen biokompatiblen Verkapselungsschichten eingebettet sind, die Polydimethylsiloxan (PDMS) umfassen.

2. Implantierbarer Stimulator nach einem vorstehenden Anspruch, wobei der implantierbare Stimulator (100, 101, 102, 103, 104, 105, 1110, 1111) weiter eine Haftschicht (1500, 1510, 1520) angrenzend an zumindest einen Teil des Substrats (300, 1400) umfasst, wobei die Haftschicht (1500, 1510, 1520) ein keramisches Material umfasst.

3. Implantierbarer Stimulator nach Anspruch 2, wobei das Substrat (300, 1400) mehr als eine angrenzende Substratschicht umfasst und die Haftschicht (1500, 1510, 1520) zwischen Substratschichten liegt.

4. Implantierbarer Stimulator nach einem der Ansprüche 2 bis 3, wobei der implantierbare Stimulator (100, 101, 102, 103, 104, 105, 1110, 1111) weiter eine oder mehrere zusätzliche Haftschichten (1500, 1510, 1520) umfasst, wobei die eine oder mehreren Haftschichten (1500, 1510, 1520) ein keramisches Material umfassen.

5. Implantierbarer Stimulator nach Anspruch 4, wobei die eine oder mehreren zusätzlichen Haftschichten (1500, 1510, 1520) zwischen Substratschichten liegen.

6. Implantierbarer Stimulator nach einem vorstehenden Anspruch, wobei das anpassbare folienartige Substrat (300, 1400) eine maximale Dicke, nahe der Elektrodenanordnung (200, 400, 1220), von 0,3 Millimeter oder von 0,2 Millimeter oder von 0,1 Millimeter aufweist.

7. Implantierbarer Stimulator nach einem vorstehenden Anspruch, wobei der Stimulator (100, 101, 102, 103, 104, 105, 1110, 1111) eine maximale Dicke, nahe dem Impulsgenerator (500), von 5 Millimeter oder von 4 Millimeter oder von 3 Millimeter aufweist.

8. Implantierbarer Stimulator nach einem vorstehenden Anspruch, wo:
das Substrat (300, 1400) eine Oberfläche aufweist, die in einer Ebene mit einer Querausdehnung enthalten ist, die im Wesentlichen senkrecht zu der Längsachse (600) vorliegt;
wobei der anpassbare Teil des Substrats (300, 1400) eine maximale planare Breite aufweist, wobei die Breite durch einen senkrechten Abstand zwischen entsprechenden Punkten auf äußeren Oberflächenkanten des Substrats (300, 1400) entlang der Querausdehnung bestimmt wird; und
wobei das Verhältnis von maximaler planarer Breite zu einer maximalen Dicke, nahe den mindestens zwei Elektroden (200, 400), gleich oder mehr als 7 : 1 ist oder gleich oder mehr als 10 : 1 ist oder gleich oder mehr als 15 : 1 ist, gleich oder mehr als 30 : 1 ist oder gleich oder mehr als 50 : 1 ist.

9. Implantierbarer Stimulator nach einem vorstehenden Anspruch, wobei der anpassbare Teil des Substrats (300, 1400) eine oder mehrere Schichten des Flüssigkristallpolymers (LCP) umfasst.

10. Implantierbarer Stimulator nach einem vorstehenden Anspruch, wobei:
- sich der Impulsgenerator (500) entlang eines ersten Teils des Substrats (300, 1400) befindet;
- sich die Elektrodenanordnung (200, 400, 1220) entlang eines zweiten anpassbaren Flüssigkristallpolymer (LCP)-Teils des Substrats (300, 1400) befindet;
wobei die Vielzahl von elektrischen Verbindungen (250, 1210) auf einer ersten anpassbaren LCP-Schicht des Substrats (300, 1400) unter Nutzung von Elektroplattierung und/oder einer Halbleiterdepositionstechnik positioniert ist und mindestens eine zweite anpassbare LCP-Schicht des Substrats (300, 1400) an der ersten Schicht angebracht ist, um die Vielzahl von elektrischen Leitungen (250, 1210) zu bedecken;
wobei der implantierbare Stimulator (100, 101, 102, 103, 104, 105, 1110, 1111) weiter Folgendes umfasst:
- eine oder mehrere Haftschichten (1500, 1510, 1520) angrenzend an das Substrat (300, 1400); und
- eine Verkapselungsschicht (1300, 1310, 1320), die den ersten Teil des Substrats (300, 1400) bedeckt, wobei die Verkapselungsschicht Polydimethylsiloxan (PDMS) umfasst;
wobei die Dicke des Substrats (300, 1400) entlang des zweiten Teils gleich oder kleiner als 0,2 Millimeter ist;
wobei eine Dicke des Stimulators entlang des ersten Teils gleich oder kleiner als 3 Millimeter ist;
wobei der Impulsgenerator (500) einen Energieempfänger umfasst, der zum drahtlosen Empfang von Energie von einem Energietransmitter konfiguriert ist.

11. Implantierbarer Stimulator nach einem vorstehenden Anspruch, wobei der Stimulator (100, 101, 102, 103, 104, 105, 1110, 1111) weiter Folgendes umfasst:
- eine Verkapselungsschicht (1300, 1310, 1320), die zumindest teilweise das Substrat (300, 1400) bedeckt; und
- eine biokompatible Haftschicht (1500, 1510, 1520) zwischen der Verkapselungsschicht (1300, 1310, 1320) und dem Substrat (300, 1400) an mindestens einer Stelle.

12. Implantierbarer Stimulator nach Anspruch 11, wobei die Verkapselungsschicht (1300, 1310, 1320) zumindest einen Teil des anpassbaren Teils des Substrats (300, 1400) bedeckt und wobei die Haftschicht (1500, 1510, 1520) zwischen der Verkapselungsschicht (1300, 1310, 1320) und dem mindestens einen Teil des anpassbaren Teils des Substrats (300, 1400) vorliegt.

13. Implantierbarer Stimulator nach einem der Ansprüche 11 bis 12, wobei die Haftschicht (1500, 1510, 1520) ein keramisches Material umfasst, wobei das keramische Material aus der Gruppe ausgewählt ist, die aus Folgenden besteht: HfO2, Al2O3, Ta2O3, Si3N4, TiO2 oder jedweder Kombination davon.

14. Implantierbarer Stimulator nach einem der Ansprüche 11 bis 13, wobei der anpassbare Teil des Substrats (300, 1400) einen Elastizitätsmodul in dem Bereich 2500 bis 3600 MPa aufweist.

15. Implantierbarer Stimulator nach einem der Ansprüche 11 bis 14, wobei die Verkapselungsschicht (1300, 1310, 1320) eine Zugfestigkeit in dem Bereich 6 bis 8 MPa aufweist.

16. Implantierbarer Stimulator nach einem der Ansprüche 11 bis 15, wobei ein keramischer Teil der Haftschicht (1500, 1510, 1520) eine durchschnittliche Dicke in dem Bereich von 25 nm bis 200 nm aufweist.

17. Stimulator nach einem der Ansprüche 1 bis 16, der zur Stimulation von Folgenden konfigurierbar ist: einem oder mehreren Nerven, einem oder mehreren Muskeln, einem oder mehreren Organen, Rückenmarksgewebe, Hirngewebe, einem oder mehreren kortikalen Oberflächenbereichen, einem oder mehreren Sulci und jedweder Kombination davon.

18. Stimulator nach einem der Ansprüche 1 bis 16, der zur Behandlung von Folgenden konfigurierbar ist: Kopfschmerzen, chronischen Kopfschmerzen, primären Kopfschmerzen, Inkontinenz, Okzipitalneuralgie, Schlafapnoe, Hypertonie, gastroösophagealer Refluxkrankheit, einer entzündlichen Erkrankung, Gliederschmerzen, Beinschmerzen, Rückenschmerzen, Schmerzen im unteren Rückenbereich, Phantomschmerz, chronischem Schmerz, Epilepsie, einer überaktiven Blase, Schmerzen nach Schlaganfall, Adipositas, einer Autoimmunerkrankung, rheumatoider Arthritis, entzündlicher Darmerkrankung, Morbus Crohn und jedweder Kombination davon.

## Revendications

1. Stimulateur implantable (100, 101, 102, 103, 104, 105, 1110, 1111) comprenant :
- un générateur d'impulsions (500) configuré pour générer au moins une impulsion de stimulation ;
- un substrat conformable de type feuille (300, 1400) présentant un axe longitudinal (600) s'étendant depuis le générateur d'impulsions (500) jusqu'à une extrémité distale du substrat (300, 1400), le substrat (300, 1400) comprenant une ou plusieurs couches de substrat polymères adjacentes, le substrat présentant des première (310, 1410) et seconde (320, 1420) surfaces sensiblement planes ; et
- un réseau d'électrodes (200, 400, 1220), à proximité de l'extrémité distale (300, 1400), présentant une première (200, 1220) et une seconde (400, 1220) électrodes comprises dans la première (310, 1410) ou la seconde surface (320, 1420), situées le long de la partie conformable du substrat (300, 1420), chaque électrode (200, 400, 1220) en fonctionnement étant configurable pour transférer une énergie de traitement, en cours d'utilisation, vers et/ou à partir de tissus humains ou animaux ;
le stimulateur implantable (100, 101, 102, 103, 104, 105, 1110, 1111) comprenant en outre :
- une ou plusieurs interconnexions électriques (250, 1210), entre le générateur d'impulsions (500) et les première (200, 1220) et deuxième (400, 1220) électrodes, pour transférer de l'énergie électrique sous forme d'une ou plusieurs impulsions de stimulation de traitement électrique aux premières électrodes (200) et/ou deuxièmes électrodes (400) couplées ;
dans lequel les une ou plusieurs interconnexions électriques (250, 1210) sont positionnées entre les première (310, 1410) et seconde (320, 1420) surfaces du substrat (300, 1400) ;
dans lequel le substrat conformable de type feuille (300, 1400) a une épaisseur maximale de 0,5 millimètre ou moins, à proximité des première et deuxième électrodes (200, 400, 1220), l'épaisseur étant déterminée par une distance perpendiculaire entre des points correspondants sur les première (310, 1410) et seconde surfaces planes externes (320, 1420), et
dans lequel le substrat (300, 1400) et le générateur d'impulsions (500) sont intégrés dans une ou plusieurs couches d'encapsulation biocompatibles flexibles comprenant du polydiméthylsiloxane (PDMS).

2. Stimulateur implantable selon l'une quelconque des revendications précédentes, le stimulateur implantable (100, 101, 102, 103, 104, 105, 1110, 1111) comprenant en outre une couche d'adhérence (1500, 1510, 1520) adjacente à au moins une partie du substrat (300, 1400), la couche d'adhérence (1500, 1510, 1520) comprenant un matériau céramique.

3. Stimulateur implantable selon la revendication 2, dans lequel le substrat (300 1400) comprend plus d'une couche de substrat adjacente et la couche d'adhérence (1500, 1510, 1520) se trouve entre des couches de substrat.

4. Stimulateur implantable selon l'une quelconque des revendications 2 à 3, le stimulateur implantable (100, 101, 102, 103, 104, 105, 1110, 1111) comprenant en outre une ou plusieurs couches d'adhérence supplémentaires (1500, 1510, 1520), les une ou plusieurs couches d'adhérence (1500, 1510, 1520) comprenant un matériau céramique.

5. Stimulateur implantable selon la revendication 4, dans lequel les une ou plusieurs couches d'adhérence supplémentaires (1500, 1510, 1520) se trouvent entre des couches de substrat.

6. Stimulateur implantable selon l'une quelconque des revendications précédentes, dans lequel le substrat conformable de type feuille (300, 1400) a une épaisseur maximale, à proximité du réseau d'électrodes (200, 400, 1220), de 0,3 millimètre, ou de 0,2 millimètre, ou de 0,1 millimètres.

7. Stimulateur implantable selon l'une quelconque des revendications précédentes, dans lequel le stimulateur (100, 101, 102, 103, 104, 105, 1110, 1111) a une épaisseur maximale, à proximité du générateur d'impulsions (500), de 5 millimètres, ou de 4 millimètres, ou de 3 millimètres.

8. Stimulateur implantable selon l'une quelconque des revendications précédentes, où :
le substrat (300, 1400) a une surface comprise dans un plan d'extension transversale sensiblement perpendiculaire à l'axe longitudinal (600) ;
dans lequel la partie conformable du substrat (300, 1400) a une largeur plane maximale, la largeur étant déterminée par une distance perpendiculaire entre des points correspondants sur des bords de surface externe du substrat (300, 1400) le long de l'étendue transversale ; et
dans lequel le rapport de la largeur plane maximale à une épaisseur maximale à proximité des au moins deux électrodes (200, 400) est égal ou supérieur à 7:1, ou est égal ou supérieur à 10:1, ou est égal ou supérieur à 15:1, est égal ou supérieur à 30:1, ou est égal ou supérieur à 50:1.

9. Stimulateur implantable selon l'une quelconque des revendications précédentes, dans lequel la partie conformable du substrat (300, 1400) comprend une ou plusieurs couches du polymère à cristaux liquides (LCP).

10. Stimulateur implantable selon l'une quelconque des revendications précédentes, dans lequel :
- le générateur d'impulsions (500) est situé le long d'une première partie du substrat (300, 1400) ;
- le réseau d'électrodes (200, 400, 1220) est situé le long d'une seconde partie de polymère à cristaux liquides (LCP) conformable du substrat (300, 1400) ;
dans lequel la pluralité d'interconnexions électriques (250, 1210) est positionnée sur une première couche de LCP conformable du substrat (300, 1400) par galvanoplastie et/ou technique de dépôt de semi-conducteur et au moins une deuxième couche de LCP conformable du substrat (300, 1400) est fixée à la première couche de manière à couvrir la pluralité d'interconnexions électriques (250, 1210) ;
le stimulateur implantable (100, 101, 102, 103, 104, 105, 1110, 1111) comprenant en outre :
- une ou plusieurs couches d'adhérence (1500, 1510, 1520) adjacentes au substrat (300, 1400) ; et
- une couche d'encapsulation (1300, 13, 10, 1320) recouvrant la première partie du substrat (300, 1400), la couche d'encapsulation comprenant du polydiméthylsiloxane (PDMS) ;
dans lequel l'épaisseur du substrat (300, 1400) le long de la deuxième partie est égale ou inférieure à 0,2 millimètre ;
dans lequel une épaisseur du stimulateur le long de la première partie est égale ou inférieure à 3 millimètres ;
dans lequel le générateur d'impulsions (500) comprend un récepteur d'énergie configuré pour recevoir sans fil de l'énergie d'un émetteur d'énergie.

11. Stimulateur implantable selon l'une quelconque des revendications précédentes, dans lequel le stimulateur (100, 101, 102, 103, 104, 105, 1110, 1111) comprend en outre :
- une couche d'encapsulation (1300, 1310, 1320) recouvrant au moins partiellement le substrat (300, 1400) ; et
- une couche d'adhérence biocompatible (1500, 1510, 1520) entre la couche d'encapsulation (1300, 13, 10, 1320) et le substrat (300, 1400) dans au moins un emplacement.

12. Stimulateur implantable selon la revendication 11, dans lequel la couche d'encapsulation (1300, 1310, 1320) recouvre au moins une partie de la partie conformable du substrat (300, 1400), et dans lequel la couche d'adhérence (1500, 1510, 1520) se trouve entre la couche d'encapsulation (1300, 1310, 1320) et l'au moins une partie de la partie conformable du substrat (300, 1400).

13. Stimulateur implantable selon l'une quelconque des revendications 11 à 12, dans lequel la couche d'adhérence (1500, 1510, 1520) comprend un matériau céramique, le matériau céramique étant sélectionné dans le groupe constitué de : HfO2, Al2O3, Ta2O3, Si3N4, TiO2, ou toute combinaison de ceux-ci.

14. Stimulateur implantable selon l'une quelconque des revendications 11 à 13, dans lequel la partie conformable du substrat (300, 1400) a un module de Young dans la plage de 2500 à 3600 MPa.

15. Stimulateur implantable selon l'une quelconque des revendications 11 à 14, dans lequel la couche d'encapsulation (1300, 1310, 1320) a une résistance à la traction dans la plage de 6 à 8 MPa.

16. Stimulateur implantable selon l'une quelconque des revendications 11 à 15, dans lequel une partie céramique de la couche d'adhérence (1500, 1510, 1520) a une épaisseur moyenne dans la plage de 25 nm à 200 nm.

17. Stimulateur selon l'une quelconque des revendications 1 à 16, configurable pour stimuler un ou plusieurs nerfs, un ou plusieurs muscles, un ou plusieurs organes, le tissu de la moelle épinière, le tissu cérébral, une ou plusieurs régions de la surface corticale, un ou plusieurs sillons, et toute combinaison de ceux-ci.

18. Stimulateur selon l'une quelconque des revendications 1 à 16, configurable pour traiter les maux de tête, les maux de tête chroniques, les maux de tête primaires, l'incontinence, la névralgie occipitale, l'apnée du sommeil, l'hypertension, le reflux gastro-oesophagien, une maladie inflammatoire, une douleur de membres, une douleur des jambes, une douleur de dos, une douleur lombaire, une douleur fantôme, une douleur chronique, l'épilepsie, une vessie hyperactive, une douleur post-AVC, l'obésité, une maladie auto-immune, la polyarthrite rhumatoïde, une maladie inflammatoire de l'intestin, la maladie de Crohn et toute combinaison de ceux-ci.
